# EUROPÄISCHE PATENTSCHRIFT

(11) **EP 4 526 055 B1**
(45) Veröffentlichungstag und Bekanntmachung des Hinweises auf die Patenterteilung: **08.07.2026**
(21) Anmeldenummer: 22729640.7
(22) Anmeldetag: 19.05.2022
(51) Int. Cl.: B06B 1/02, A61B 8/00, G01H 11/06, G01S 7/52

(54) **ULTRASCHALLSYSTEM SOWIE VERFAHREN ZUR MODIFIKATION EINES DER MEHRZAHL AN MODULAREN ULTRASCHALLGERÄTEN IN DEM ULTRASCHALLSYSTEM**
ULTRASOUND SYSTEM AND METHOD FOR MODIFYING ONE OF THE PLURALITY OF MODULAR ULTRASOUND DEVICES IN THE ULTRASOUND SYSTEM
SYSTÈME À ULTRASONS ET PROCÉDÉ DE MODIFICATION D'UN DES APPAREILS À ULTRASONS MODULAIRES DANS LE SYSTÈME À ULTRASONS

(43) Veröffentlichungstag der Anmeldung: 26.03.2025
(73) Patentinhaber: Fraunhofer-Gesellschaft zur Förderung der angewandten Forschung e.V., 80686 München (DE)
(72) Erfinder: HEWENER, Holger, 66280 Sulzbach (DE); TRETBAR, Steffen, 66280 Sulzbach (DE)
(74) Vertreter: 2SPL Patentanwälte PartG mbB
(86) Internationale Anmeldenummer: PCT/EP2022/063541
(87) Internationale Veröffentlichungsnummer: WO 2023/222225

(56) Entgegenhaltungen:
- WO-A1-2014/145007
- CN-A- 112 807 015
- US-A1- 2011 059 660
- US-A1- 2013 064 037

## Beschreibung

### Technisches Gebiet

Die vorliegende Offenbarung befasst sich mit der Anwendung von Ultraschall. Insbesondere betreffen Ausführungsbeispiele ein Ultraschallsystem sowie ein Verfahren zur Modifikation eines der Mehrzahl an modularen Ultraschallgeräten in dem Ultraschallsystem.

### Hintergrund

Ultraschall ist eine in vielen Bereichen der Medizin, aber auch in der industriellen Prüf- und Messtechnik eine etablierte, kostengünstige, echtzeitfähige, nicht-invasive und überall einsetzbare Methode. Für die sehr große Bandbreite aller mit Ultraschall möglichen Anwendungen stehen verschiedenste Systeme unterschiedlicher Integrationsgrade und Komplexitätsstufen zur Verfügung. Allerdings sind alle am Markt befindlichen Systeme dedizierte, geschlossene und für nur wenige Anwendungsfälle abgestimmte Entwicklungen, die ihre "spezifische Intelligenz" ausschließlich in einer Schaltungslogik eingebettet in der Hardware tragen oder von Seiten der Hardware entsprechend eingeschränkt sind. Dieser Umstand macht alle diese Systeme unflexibel, bedingt hohe Entwicklungskosten für neue Anwendungen (Anforderungen) sowie eine relativ lange "Time-to-Market" beim Transfer der Ultraschalltechnologie.

Gleichzeitig erfahren mobile, "persönliche" Endgeräte (z.B. Smartphones, Tablets, etc.) in den letzten Jahren eine starke Marktdurchdringung. Getrieben durch den breiten Massenmarkt im sog. "Consumer-Bereich" steigen sowohl die Leistungsfähigkeit dieser Geräte als auch deren Einsatzmöglichkeiten. Diese Entwicklung wird seit einigen Jahren von verschiedenen Herstellern erkannt und es werden erste Geräte realisiert, die auf Consumer-Elektronik basieren. Allerdings sind diese Geräte immer für einen speziellen Anwendungsfall entwickelt, sind relativ groß und nutzen das Endgerät lediglich als kompaktes User-Interface oder als Schnittstelle zu einer Cloud.

Zusammenfassend ist festzustellen, dass existierende Geräte jeweils nur ein spezifisches oder sehr enges Anwendungsgebiet abdecken und nur wenige mobil einsetzbar sind.

Dokument US 2013/064037 A1 beschreibt ein Verfahren und eine Vorrichtung zur Erfassung von Ultraschallbildern. Die Vorrichtung ist in das Gehäuse einer Ultraschallsonde integriert, das eine Anordnung elektroakustischer Wandler umfasst, die Ultraschallimpulse senden und empfangen. Diese Anordnung ist mit einer Verarbeitungseinheit verbunden, die die Empfangssignale verarbeitet und Signale zur Erzeugung von Ultraschallwellen sendet. Der Verarbeitungseinheit, die in das Sondengehäuse eingebaut ist, wandelt Empfangssignale in Bilder um und erzeugt Videosignale zur Bilddarstellung auf einem Display. Die Übertragung zwischen der Sonde und einer entfernten Einheit zur Anzeige und Speicherung der Bilder als Videosignale kann kabellos erfolgen.

Vor diesem Hintergrund ist es eine Aufgabe, eine verbesserte Anwendung und flexiblere Bereitstellung von Ultraschall zu ermöglichen.

### Zusammenfassung

Die Aufgabe wird erfindungsgemäß durch ein Ultraschallsystem sowie ein Verfahren zur Modifikation eines der Mehrzahl an modularen Ultraschallgeräten in dem Ultraschallsystem gemäß den unabhängigen Ansprüchen gelöst. Weitere Aspekte sowie Weiterbildungen der Erfindung sind in den abhängigen Ansprüchen, der folgenden Beschreibung sowie in den Figuren beschrieben.

Ein erstes Ausführungsbeispiel betrifft ein Ultraschallsystem. Das Ultraschallsystem umfasst eine Mehrzahl an erfindungsgemäßen modularen Ultraschallgeräten und eine Vorrichtung zur Auswertung von Messdaten. Die Mehrzahl an modularen Ultraschallgeräten sind ausgebildet, jeweilige Messdaten direkt oder durch Weiterleitung über zumindest ein weiteres der Mehrzahl an modularen Ultraschallgeräten zu einem oder mehreren vorbestimmten modularen Ultraschallgeräten der Mehrzahl an modularen Ultraschallgeräten zu senden. Das eine oder die mehreren vorbestimmten modularen Ultraschallgeräten ist ausgebildet, die gesammelten Messdaten der Mehrzahl an modularen Ultraschallgeräten an die Vorrichtung zur Auswertung von Messdaten zu senden. Die Vorrichtung zur Auswertung von Messdaten ist ausgebildet, eine oder mehrere vorbestimmte Kenngrößen basierend auf den Messdaten zu bestimmen. Die Mehrzahl an modularen Ultraschallgeräten umfasst jeweils ein Gehäuse und zumindest einen Ultraschallwandler. Der zumindest eine Ultraschallwandler ist ausgebildet, basierend auf einem jeweiligen Steuerungssignal Ultraschallwellen zu erzeugen und auszusenden und abhängig von empfangenen Ultraschallwellen ein jeweiliges Messsignal zu erzeugen. Die Mehrzahl an modularen Ultraschallgeräten umfasst jeweils umfasst weiterhin eine Mehrzahl an in dem Gehäuse angeordneten Platinen, die über jeweilige Steckverbinder trennbar miteinander verbunden sind. Die Mehrzahl an Platinen umfasst zumindest eine erste Platine mit einer Stromversorgungsschaltung, die ausgebildet ist, ein jeweiliges Stromversorgungssignal für die weiteren Platinen der Mehrzahl an Platinen zu erzeugen. Die Mehrzahl an Platinen umfasst ferner zumindest eine Sendeschaltung, die ausgebildet ist, das jeweilige Steuerungssignale für den zumindest einen Ultraschallwandler zu erzeugen, sowie eine Empfangsschaltung, die ausgebildet ist, das jeweilige Messsignale des zumindest einen Ultraschallwandlers zu verarbeiten. Die Sendeschaltung und die Empfangsschaltung sind dabei entweder beide auf einer zweiten Platine der Mehrzahl an Platinen ausgebildet oder die Sendeschaltung ist auf der zweiten Platine und die Empfangsschaltung ist auf einer dritten Platine der Mehrzahl an Platinen ausgebildet. Der zumindest eine Ultraschallwandler ist auf einer vierten Platine der Mehrzahl an Platinen ausgebildet oder trennbar mit einer der Mehrzahl an Platinen verbunden. Die Mehrzahl an Platinen umfasst ferner eine fünfte Platine mit einer Sendeempfängerschaltung, die eingerichtet ist, basierend auf zu übertragenden Daten des mobilen Ultraschallgeräts ein Sendesignal zu erzeugen. Die Sendeempfängerschaltung ist ferner eingerichtet, basierend auf einem Empfangssignal Empfangsdaten für das mobile Ultraschallgerät zu bestimmen. Zudem ist die Sendeempfängerschaltung eingerichtet, das Sendesignal an eine Antenne des modularen Ultraschallgeräts zur Abstrahlung in eine Umgebung des modularen Ultraschallgeräts anzulegen und/oder das Sendesignal an eine Schnittstelle des mobilen Ultraschallgeräts zur drahtgebundenen Kommunikation mit einem externen Gerät anzulegen und/oder das Sendesignal an die Sendeschaltung weiterzuleiten, um das jeweilige Steuerungssignal für den zumindest einen Ultraschallwandler basierend auf dem Sendesignal zu erzeugen und so die zu übertragenden Daten des mobilen Ultraschallgeräts in die von dem zumindest einen Ultraschallwandler ausgesendeten Ultraschallwellen zu kodieren. Weiterhin ist die Sendeempfängerschaltung eingerichtet, das Empfangssignal von der Antenne zu empfangen und/oder das Empfangssignal von der Schnittstelle zur drahtgebundenen Kommunikation mit dem externen Gerät zu empfangen und/oder das Empfangssignal von der Empfangsschaltung zu empfangen. Die Empfangsschaltung ist dabei eingerichtet, das Empfangssignal aus dem jeweiligen Messsignal des zumindest einen Ultraschallwandlers abzuleiten.

Ein zweites Ausführungsbeispiel betrifft ein Verfahren zur Modifikation eines der Mehrzahl an modularen Ultraschallgeräten in dem erfindungsgemäßen Ultraschallsystem. Das Verfahren umfasst ein zerstörungsfreies Trennen einer der Mehrzahl an Platinen von den übrigen Platinen der Mehrzahl an Platinen durch Lösen zumindest eines Teils der Steckverbinder. Zudem umfasst das Verfahren ein trennbares Verbinden einer neuen Platine mit den übrigen Platinen der Mehrzahl an Platinen mittels eines oder mehrerer Steckverbinder.

### Figurenkurzbeschreibung

Einige Beispiele von Vorrichtungen und/oder Verfahren werden nachfolgend bezugnehmend auf die beiliegenden Figuren lediglich beispielhaft näher erläutert. Es zeigen:
Fig. 1 eine dreidimensionale Darstellung eines erste Ausführungsbeispiels eines modularen Ultraschallgeräts;
Fig. 2 eine Explosionsdarstellung des in Fig. 1 gezeigten modularen Ultraschallgeräts;
Fig. 3 eine schematische Darstellung eines zweiten Ausführungsbeispiels eines modularen Ultraschallgeräts;
Fig. 4 eine schematische Darstellung eines dritten Ausführungsbeispiels eines modularen Ultraschallgeräts;
Fig. 5a und Fig. 5b eine schematische Darstellung eines vierten Ausführungsbeispiels eines modularen Ultraschallgeräts, das mit Zusatzgeräten gekoppelt ist;
Fig. 6 eine schematische Darstellung eines ersten Ausführungsbeispiels eines Ultraschallsystems;
Fig. 7 eine schematische Darstellung eines zweiten Ausführungsbeispiels eines Ultraschallsystems;
Fig. 8 eine schematische Darstellung eines dritten Ausführungsbeispiels eines Ultraschallsystems;
Fig. 9 ein Anwendungsszenario für das in Fig. 8 gezeigten Ultraschallsystems; und
Fig. 10 ein Ablaufdiagramm eines Ausführungsbeispiels eines Verfahrens zur Modifikation eines modularen Ultraschallgeräts.

### Beschreibung

Einige Beispiele werden nun ausführlicher Bezug nehmend auf die beiliegenden Figuren beschrieben. Weitere mögliche Beispiele sind jedoch nicht auf die Merkmale dieser detailliert beschriebenen Ausführungsformen beschränkt. Diese können Modifikationen der Merkmale sowie Entsprechungen und Alternativen zu den Merkmalen aufweisen. Ferner soll die Terminologie, die hierin zum Beschreiben bestimmter Beispiele verwendet wird, nicht einschränkend für weitere mögliche Beispiele sein.

Gleiche oder ähnliche Bezugszeichen beziehen sich in der gesamten Beschreibung der Figuren auf gleiche oder ähnliche Elemente beziehungsweise Merkmale, die jeweils identisch oder auch in abgewandelter Form implementiert sein können, während sie die gleiche oder eine ähnliche Funktion bereitstellen. In den Figuren können ferner die Stärken von Linien, Schichten und/oder Bereichen zur Verdeutlichung übertrieben sein.

Wenn zwei Elemente A und B unter Verwendung eines "oder" kombiniert werden, ist dies so zu verstehen, dass alle möglichen Kombinationen offenbart sind, d. h. nur A, nur B sowie A und B, sofern nicht im Einzelfall ausdrücklich anders definiert. Als alternative Formulierung für die gleichen Kombinationen kann "zumindest eines von A und B" oder "A und/oder B" verwendet werden. Das gilt Äquivalent für Kombinationen von mehr als zwei Elementen.

Wenn eine Singularform, z. B. "ein, eine" und "der, die, das" verwendet wird und die Verwendung nur eines einzelnen Elements weder explizit noch implizit als verpflichtend definiert ist, können weitere Beispiele auch mehrere Elemente verwenden, um die gleiche Funktion zu implementieren. Wenn eine Funktion im Folgenden als unter Verwendung mehrerer Elemente implementiert beschrieben ist, können weitere Beispiele die gleiche Funktion unter Verwendung eines einzelnen Elements oder einer einzelnen Verarbeitungsentität implementieren. Es versteht sich weiterhin, dass die Begriffe "umfasst", "umfassend", "aufweist" und/oder "aufweisend" bei deren Gebrauch das Vorhandensein der angegebenen Merkmale, Ganzzahlen, Schritte, Operationen, Prozesse, Elemente, Komponenten und/oder einer Gruppe derselben beschreiben, dabei aber nicht das Vorhandensein oder das Hinzufügen eines oder mehrerer anderer Merkmale, Ganzzahlen, Schritte, Operationen, Prozesse, Elemente, Komponenten und/einer Gruppe derselben ausschließen.

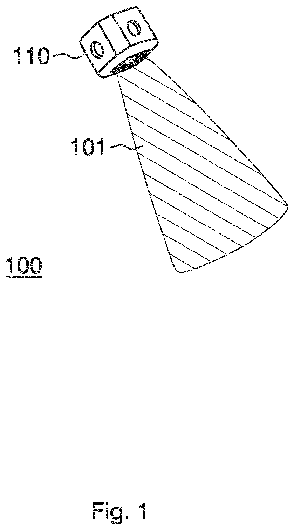

Fig. 1 zeigt ein modulares Ultraschallgerät 100 gemäß der vorliegenden Offenbarung. Das modulare Ultraschallgerät 100 umfasst ein Gehäuse 110, in dem die diversen Komponenten des modularen Ultraschallgeräts 100 untergebracht sind. Das modulare Ultraschallgerät 100 ist ausgebildet, Ultraschallwellen 101 auszusenden.

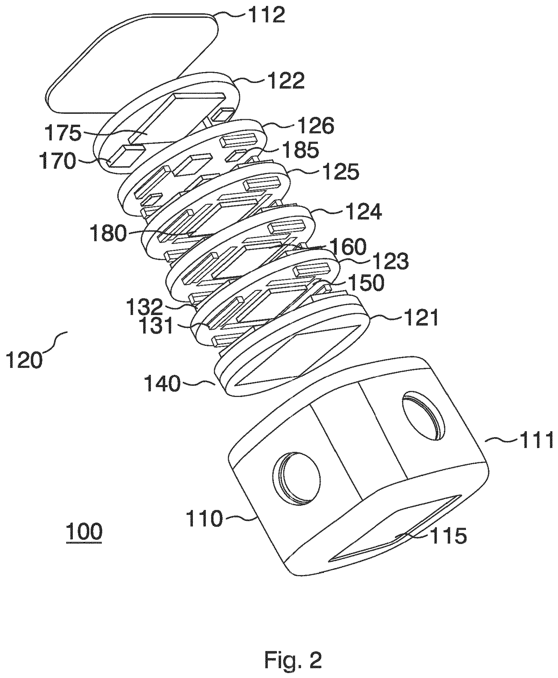

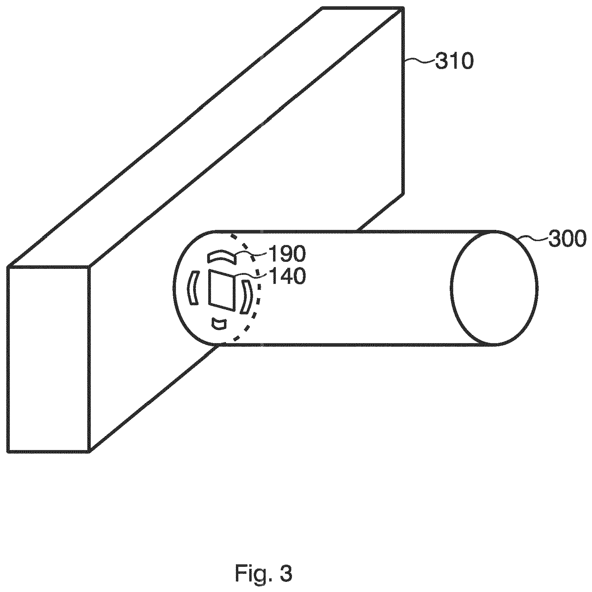

Eine Explosionsdarstellung des modularen Ultraschallgeräts 100, in dem die einzelnen Komponenten des modularen Ultraschallgeräts 100 erkennbar sind, ist in Fig. 2 gezeigt. Das Gehäuse 110 des modularen Ultraschallgeräts 100 besteht aus zwei Teilen 111 und 112, welche miteinander verbindbar und zerstörungsfrei voneinander trennbar sind, so dass die diversen Komponenten innerhalb des modularen Ultraschallgeräts 100 zugänglich bzw. erreichbar sind. Es ist jedoch zu beachten, dass das Gehäuse 110 des modularen Ultraschallgeräts 100 nicht zwangsläufig aus zwei Teilen bestehen muss. Gemäß der vorliegenden Offenbarung kann das Gehäuse 110 auch aus mehr als zwei Teilen, die miteinander verbindbar und zerstörungsfrei voneinander trennbar sind, bestehen. Im Ausführungsbeispiel der Figs. 1 und 2 weist das Gehäuse 110 eine im Wesentlichen kubische Form mit abgerundeten Kanten auf. Es ist jedoch zu beachten, dass das Gehäuse 110 des modularen Ultraschallgeräts 100 nicht zwangsläufig eine im Wesentlichen kubische Form mit abgerundeten Kanten aufweisen muss. Grundsätzlich kann das Gehäuse 110 jegliche geeignete Form aufweisen (siehe z.B. das Ausführungsbeispiel der Fig. 3).

Wie aus Fig. 2 ersichtlich ist, umfasst das modulare Ultraschallgerät 100 eine Mehrzahl an Platinen 120, die in dem Gehäuse 110 angeordnet sind. Die Mehrzahl an Platinen 120 sind über jeweilige Steckverbinder 131, 132 trennbar miteinander verbunden. Das Teil 112 des Gehäuses 110 kann z.B. als eine Gehäuserückseite mit einem Mechanismus zum Schließen (z.B. Schraub - oder Klemmmechanismus) konzipiert sein, um die Mehrzahl an Platinen 120 erreichen bzw. in das Gehäuse 110 einbringen zu können.

Das modulare Ultraschallgerät 100 umfasst zumindest einem Ultraschallwandler 140, der ausgebildet ist, basierend auf einem jeweiligen Steuerungssignal die Ultraschallwellen 101 zu erzeugen und auszusenden. Ferner ist der zumindest einem Ultraschallwandler 140 ausgebildet, abhängig von empfangenen Ultraschallwellen ein jeweiliges Messsignal zu erzeugen. Im Ausführungsbeispiel der Fig. 2 ist der zumindest eine Ultraschallwandler 140 auf einer ersten Platine 121 der Mehrzahl an Platinen 120 ausgebildet. Gemäß einigen Ausführungsbeispielen kann die erste Platine 121 auch eine Mehrzahl an Ultraschallwandlern 140 (d.h. zwei oder mehr), die wie vorbeschrieben ausgebildet sind, aufweisen.

Allgemein können verschiedene Geometrien und Anordnungen von als Ultraschallwandler dienenden akustischen Elementen (z.B. Einelementwandler mit und ohne strukturierte Linsen zur Volumenbildgebung, Arraywandler in Linear-, Konvex, Konkav, T- und Matrix-Geometrie oder beliebiger Form und Elementanordnung) mit unterschiedlichen Eigenschaften (schmalbandig, breitbandig, sensitiv) als Sender oder als Empfänger oder beides oder nur teilweise umgesetzt und kombiniert werden. Somit können Parameter wie der anwendungsspezifische Frequenzbereich, die Ausgangsleistung, die Anzahl der Kanäle, die Ultraschall-Geometrie, etc. sehr einfach und flexibel über die standardisierten Hardware-Schnittstellen variiert werden. Ein weiter Frequenzbereich ist realisierbar (Hz-, kHz-, bis MHz-Bereich) vom Hörschall über Luftschall bis Körperschall von Flüssigkeiten bis Feststoffen. Mit dieser Bandbreite an Typen möglicher Ultraschallwandler kann somit eine große Bandbreite von punktuellen Messungen, einfacher Bildgebung bis hin zu volumetrischer Erfassung durchgeführt werden.

Die erste Platine 121 kann als ein Ultraschallwandler-Modul des modularen Ultraschallgeräts 100 aufgefasst werden. Dabei ist jedoch zu beachten, dass der zumindest eine Ultraschallwandler 140 nicht bzw. nicht jeder Ultraschallwandler bei einer Mehrzahl an Ultraschallwandlern auf einer der Mehrzahl an Platinen 120 ausgebildet sein muss. In alternativen Ausführungsbeispielen kann der zumindest eine Ultraschallwandler 140 auch separat von der Mehrzahl an Platinen 120 in dem Gehäuse 110 angeordnet und z.B. trennbar mit einer der Mehrzahl an Platinen 120 verbunden sein (z.B. über eines oder mehrere Kabel).

Das Gehäuse 110 umfasst ein Fenster mit gegenüber dem sonstigen Gehäuse erhöhter akustischer Transparenz. Das Fenster 115 ist vor dem zumindest einen Ultraschallwandler 140 angeordnet. Im Beispiel der Fig. 2 ist das Fenster 115 durch eine Aussparung (Öffnung) in dem Gehäuse 110 gebildet. Es ist jedoch zu beachten, dass das Fenster 115 nicht zwangsläufig durch eine Aussparung im Gehäuse 110 gebildet sein muss. Vielmehr kann das Gehäuse in einem Teilbereich vor dem zumindest einen Ultraschallwandler 140 auch aus einem ersten Material oder einer ersten Materialmischung gebildet sein, das/die gegenüber einem zweiten Material oder einer zweiten Materialmischung des sonstigen Gehäuses eine angepasste oder erhöhte akustischer Transparenz aufweist.

Ferner umfasst die Mehrzahl an Platinen 120 zumindest eine zweite Platine 122 mit einer Stromversorgungsschaltung 170, die ausgebildet ist, ein jeweiliges Stromversorgungssignal für den zumindest einen Ultraschallwandler und die weiteren Platinen der Mehrzahl an Platinen 120 zu erzeugen. Weiterhin kann die Stromversorgungsschaltung 170 für das bedarfsgerechte Energiemanagement des modularen Ultraschallgeräts 100 ausgebildet sein. Beispielsweise kann die Stromversorgungsschaltung 170 ausgebildet sein, eine oder mehrere der Mehrzahl an Platinen 120 oder einzelne Komponenten auf der Mehrzahl an Platinen während z.B. Langzeitmessaufgaben zumindest teilweise in einen Energiesparmodus (z.B. einen Sleep-Modus) zu versetzen. Die Stromversorgungsschaltung 170 oder eine sonstige Schaltung (z.B. eine nachfolgend noch näher beschriebene Prozessierschaltung) kann den Sleep-Mode zusätzlich adaptiv je nach vorherigen Messungen und derer Ergebnisse autonom parametrisieren, um noch effizienter Langzeitmessungen durchzuführen. Ein Faktor, von dem dies beispielsweise abhängig gemacht werden kann, ist das baldige Erreichen eines Grenzwertes, den es zu überwachen gilt, zur Verkürzung von Messintervallen (z.B. maximaler oder minimaler Füllstand) oder der Verlängerung des Messintervalls, wenn der Messwert sich weit von entscheidenden Grenzen befindet.

Optional kann die zweite Platine 120 ferner einen mit der Stromversorgungsschaltung 170 gekoppelten Akkumulator 175 umfassen. Entsprechend kann die Stromversorgungsschaltung 170 ausgebildet sein, das jeweilige Stromversorgungssignal basierend auf in dem Akkumulator 175 gespeicherter Energie zu erzeugen. Der Akkumulator 175 kann auch austauschbar gestaltet sein. Beispielsweise kann die Speicherkapazität des Akkumulators 175 je nach Einsatzzweck und Anwendung gewählt sein. Die Stromversorgungsschaltung 170 übernimmt auch das Lademanagement für den Akkumulator 175 (abhängig von der gewählten Energiequelle, siehe unten).

Das modulare Ultraschallgerät 100 kann optional mehrere zweite Platinen 122 umfassen, um die Gesamtkapazität der verfügbaren elektrischen Energie zu erhöhen. Die Gesamtkapazität der verfügbaren elektrischen Energie ist dann nicht durch Kapazität des Akkumulators auf der einzelnen Platine definiert, sondern skalierbar durch Nutzung mehrerer zweiter Platinen 122. Beispielsweise kann nur die Stromversorgungsschaltung 170 einer einzelnen der mehreren zweiten Platinen mit der oben beschriebenen Ladefunktion integriert sein und ausgebildet sein, die Akkumulatoren der weiteren zweiten Platinen z.B. mittels induktiver Kopplung zu laden.

Alternativ oder ergänzend kann in das Gehäuse 110 eine Buchse (in Figs. 1 und 2 nicht dargestellt) für die Verbindung mit einem Ladekabel integriert sein. Entsprechend kann die Stromversorgungsschaltung 170 ausgebildet sein, das jeweilige Stromversorgungssignal basierend auf an der Buchse empfangener elektrischer Energie zu erzeugen. Die Buchse kann sowohl proprietär als auch gemäß einem Industriestandard (z.B. Hochbuchsen zur Nutzung weit verbreiteter Stecker- und Tisch-Netzteile) ausgeführt sein. Die Stromversorgung kann auch "Bus-powered" erfolgen, wobei das System von außen über einen eingesetzten Kommunikationskanal parallel mit Strom versorgt wird. Dies kann z.B. mittels Power over Ethernet (PoE) bei Nutzung von Local Area Network (LAN) / Ethernet oder über Universal Serial Bus (USB) erfolgen, wobei das jeweilige Datenkabel gleichzeitig als Ladekabel fungiert.

Ferner alternativ oder ergänzend kann das modulare Ultraschallgerät 100 einen Energiewandler (in Figs. 1 und 2 nicht dargestellt) umfassen, der eingerichtet ist, Umgebungsenergie aus der Umgebung des modularen Ultraschallgeräts 120 in elektrische Energie zu wandeln. Entsprechend kann die Stromversorgungsschaltung 170 ausgebildet sein, das jeweilige Stromversorgungssignal basierend auf der durch den Energiewandler bereitgestellten elektrischen Energie zu erzeugen. Beispielsweise kann der Energiewandler in Form eines an dem Gehäuse angebrachten photoelektrischen Wandlers wie einer oder mehrerer Solarzellen bereitgestellt sein. Alternativ oder ergänzend kann der Energiewandler in Form eines thermoelektrischen Wandlers (z.B. unter Nutzung des Seebeck-Effekts, des Peltier-Effekts und/oder des Thompson-Effekts) bereitgestellt sein. Ferner alternativ oder ergänzend kann der Energiewandler in Form einer oder mehrerer Spulen bereitgestellt werden, um in der Umgebung des modularen Ultraschallgeräts 120 bereitgestellte elektromagnetische Energie mittels Induktion in elektrische Energie zu wandeln (z.B. gemäß einer proprietären Energieübertragung oder gemäß einem Industriestandard wie "Qi"). Alternativ oder ergänzend kann der Energiewandler in Form eines elektromechanischen Wandlers (z.B. unter Nutzung des Piezo-Effekts) bereitgestellt werden, um in der Umgebung des modularen Ultraschallgeräts 120 vorhandene mechanische Energie (z.B. Schwingungen, Vibrationen, Drücke oder Ultraschallwellen) in elektrische Energie zu wandeln. Derart kann eine vollständige oder zusätzlich kabellose Energieversorgung des modularen Ultraschallgeräts 100 ermöglicht werden. Wie in Fig. 2 gezeigt, kann die zweite Platine 122 z.B. die letzte Platine der Mehrzahl an Platinen 120 sein und das Gehäuseteil 112 an den Energiewandler angepasst sein, um eine optimale Nutzung der Umgebungsenergie zu ermöglichen.

Wie die obigen Beispiele gezeigt haben, kann die Versorgung mit elektrischer Energie kabelgebunden und/oder kabellos erfolgen. Sowohl Gleichstrom- als auch Wechselstromquellen können genutzt werden. Bei einer Wechselstromquelle kann beispielsweise die Stromversorgungsschaltung 170 die notwendige Gleichrichtung des Wechselstroms in Gleichstrom durchführen, um die Elektronik des modularen Ultraschallgeräts 100 mit Gleichstrom versorgen zu können.

Das modulare Ultraschallgerät 100 umfasst ferner zumindest eine Sendeschaltung 150, die ausgebildet ist, das jeweilige Steuerungssignale für den zumindest einen Ultraschallwandler 140 zu erzeugen, sowie eine Empfangsschaltung 160, die ausgebildet ist, das jeweilige Messsignale des zumindest einen Ultraschallwandlers 140 zu verarbeiten. Im Ausführungsbeispiel der Figs. 1 und 2 ist die Sendeschaltung 150 ist auf einer dritten Platine der Mehrzahl an Platinen 120 und die Empfangsschaltung ist auf einer vierten Platine 124 der Mehrzahl an Platinen 120 ausgebildet. Mit anderen Worten: Die Sendeschaltung 150 und die Empfangsschaltung 160 sind auf verschiedenen Platinen angeordnet. Es ist jedoch zu beachten, dass die Sendeschaltung 150 und die Empfangsschaltung 160 nicht zwangsläufig auf verschiedenen Platinen angeordnet sein müssen. In alternativen Ausführungsbeispielen können die Sendeschaltung 150 und die Empfangsschaltung 160 auch auf derselben Platine angeordnet sein. Beispielsweise können die Sendeschaltung 150 und die Empfangsschaltung 160 gemeinsam auf der dritten Platine 123 angeordnet sein. Wenn die Sendeschaltung 150 und die Empfangsschaltung 160 auf derselben Platine angeordnet sind, können die Sendeschaltung 150 und die Empfangsschaltung 160 auch als integrierte Schaltung, d.h. als eine Sendeempfängerschaltung ausgeführt sein.

Die Sendeschaltung 150 ist ausgebildet, die elektrischen Sendemuster und Impulse zur Ansteuerung des zumindest einen Ultraschallwandlers 140 zu erzeugen und auf ein entsprechendes Spannungsniveau zu bringen. Dies kann zumindest eines von Phasen-, Frequenz-, Amplituden- und Puls-Weiten-Modulationen umfassen. Das jeweilige Steuerungssignal für den zumindest einen Ultraschallwandler 140 gibt entsprechend die elektrischen Sendemuster und Impulse zur Ansteuerung des zumindest einen Ultraschallwandlers 140 an. Die dritte Platine 123 mit der Sendeschaltung 150 kann als ein Ultraschallelektronik-Modul zum Senden aufgefasst werden.

Die Empfangsschaltung 160 ist ausgebildet, das jeweilige Messsignal des zumindest einen Ultraschallwandlers 140 zu verarbeiten bzw. vorzuprozessieren. Dazu kann die Empfangsschaltung 160 zumindest ausgebildet sein, das jeweilige Messsignal des zumindest einen Ultraschallwandlers 140 zumindest zu verstärken und zu digitalisieren. Optional kann die Empfangsschaltung 160 auch ausgebildet sein, das jeweilige Messsignal des zumindest einen Ultraschallwandlers 140 weiter zu prozessieren. Beispielsweise kann die Empfangsschaltung 160 ferner ausgebildet sein, das jeweilige Messsignal des zumindest einen Ultraschallwandlers 140 zu komprimieren, zu filtern, mit einem anderen Signal zu addieren (z.B. die Messsignale mehrerer Ultraschallwandler zu addieren). Die vierte Platine 124 mit der Empfangsschaltung 160 kann als ein Ultraschallelektronik-Modul zum Empfangen aufgefasst werden.

Eine Konfiguration der Mehrzahl an Platinen 120, die zumindest die erste Platine 121, die zweite Platine 122 als auch die Sendeschaltung 150 und die Empfangsschaltung 160 - unabhängig davon, ob diese auf einer gemeinsamen Platine oder separaten Platinen angeordnet sind - umfasst, kann als eine Grundkonfiguration des vorgeschlagenen modularen Ultraschallgeräts aufgefasst. Sämtliche weiteren der vorangehend und nachfolgend beschriebenen Komponenten bzw. Funktionalitäten des modularen Ultraschallgeräts sind optional. Wenn der zumindest eine Ultraschallwandler 140 nicht auf einer der Mehrzahl an Platinen 120 ausgebildet ist, kann entsprechend der zumindest eine Ultraschallwandler 140 zusammen mit einer Konfiguration der Mehrzahl an Platinen 120, die zumindest die zweite Platine 122 als auch die Sendeschaltung 150 und die Empfangsschaltung 160 - unabhängig davon, ob diese auf einer gemeinsamen Platine oder separaten Platinen angeordnet sind - umfasst, als eine Grundkonfiguration des vorgeschlagenen modularen Ultraschallgeräts aufgefasst werden. Der zumindest eine Ultraschallwandler 140 kann beispielsweise mit der bzw. den Platinen, auf der bzw. denen die Sendeschaltung 150 und die Empfangsschaltung 160 ausgebildet sind, trennbar verbunden sein (z.B. mittels eines oder mehrerer Kabel).

Beispielsweise kann die Mehrzahl an Platinen ferner eine sechste Platine 125 mit einer Prozessierschaltung 180 umfassen. Die Prozessierschaltung 180 ist ausgebildet, eine jeweilige Funktionalität der Mehrzahl an Platinen 120 zu erkennen und basierend darauf einen Datenaustausch zwischen zumindest einem Teil der Mehrzahl an Platinen 120 (oder ggf. allen der Mehrzahl an Platinen 120) zu steuern. Durch die Prozessierschaltung 180 erfolgt die Steuerung des modularen Ultraschallgeräts 100. Erkennung und Kontrolle der einzelnen Module bzw. Platinen als auch Steuerung der Kommunikation und Datenaustausch der Module bzw. Platinen untereinander kann auf vielfältige Weise erfolgen und ist an sich jeweils bekannt. Daher wird an dieser Stelle darauf nicht näher eingegangen.

Beispielsweise kann die gesamte Konfiguration des modularen Ultraschallgeräts 100 während der Inbetriebnahme und Fertigung des Geräts in die Module bzw. Platinen hineinkonfiguriert werden. Auf diese Weise sind den einzelnen Modulen bzw. Platinen die vorhandenen Komponenten bekannt und Fehler oder Ausfälle anderer Module können von den verbleibenden Modulen erkannt werden. Auf diese Weise kann ggf. mittels eines oder mehrerer Notbetriebsmodi ein weiterer Messbetrieb trotz Teildefekt und -ausfall durch automatische Rekonfiguration durch das modulare Ultraschallgerät 100 selbst fortgeführt werden.

Für die Prozessierschaltung 180 kann unterschiedlich leistungsstarke und somit unterschiedlich kostenintensive Hardware genutzt werden - je nach gewünschter Anwendung des modularen Ultraschallgeräts 100. So kann die sechste Platine 125 an die benötigte Leistungsfähigkeit angepasst werden. Beispielsweise kann die Prozessierschaltung 180 durch einen Prozessor, einen Computerprozessor (engl. CPU = Central Processing Unit), einen Mikrocontroller, einen anwendungsspezifischen integrierten Schaltkreis (engl. ASIC = Application-Specific Integrated Circuit), einen integrierten Schaltkreis (engl. IC = Integrated Circuit), ein Ein-Chip-System (engl. SoC = System on a Chip), ein programmierbares Logikelement oder ein feldprogrammierbares Gatterarray (FPGA = Field Programmable Gate Array) mit einem Mikroprozessor gebildet sein bzw. aufweisen, auf dem/der Software für die Steuerung der einen bzw. der mehreren Bestandteile des modularen Ultraschallgeräts 100 gemäß den hierin beschriebenen Grundsätzen abläuft. Ferner kann die Prozessierschaltung 180 einen oder mehrere Speicher aufweisen bzw. mit diesen gekoppelt sein.

Beispielsweise kann die Prozessierschaltung 180 leistungsfähiger ausgelegt sein, um z.B. Datenverarbeitungsschritte durchzuführen, Datenanalysen durchzuführen oder Künstliche Intelligenz (KI)-Netzwerke zu nutzen. Mit anderen Worten: Die Prozessierschaltung 180 kann optional ausgebildet sein, eine oder mehrere vorbestimmte Kenngrößen basierend auf dem durch die Empfangsschaltung 160 verarbeiteten jeweiligen Messsignal des zumindest einen Ultraschallwandlers 140 zu bestimmen. Insbesondere kann die Prozessierschaltung 180 ausgebildet sein, die eine oder die mehreren vorbestimmten Kenngrößen unter Verwendung eines mittels maschinellen Lernens trainierten Modells zu bestimmen. Jedoch muss kein mittels maschinellen Lernens trainierten Modells dazu genutzt werden. Das maschinelle Lernen kann bspw. auf Hochfrequenz- (HF-) Daten erfolgen, wie sie direkt nach der Ultraschalldigitalisierung durch die Empfangsschaltung 160 vorliegen. Für die Verarbeitung der Ultraschalldaten können diese beispielsweise als Zeitseriendaten (engl. time series data) betrachtet werden. Für die Verarbeitung kann die Klassifikation im Sinne einer "time series classification" stattfinden. Hierfür und für andere Arten der Weiterverarbeitung kann die Bestimmung bzw. Extraktion von Kenngrößeren (Features) beispielsweise temporale, spektrale als auch statistische Verfahren nutzen.

Die sechste Platine 125 kann als ein Ultraschallelektronik-Modul mit Steuerlogik aufgefasst werden.

Die Prozessierschaltung 180 kann auch als Multiplexer-Modul fungieren, um eine programmierbare, dynamische und/oder feste Verbindung zwischen einzelnen Elementen der ersten Platine 121 (z.B. einzelnen Ultraschallwandlern, wenn eine Mehrzahl an Ultraschallwandlern auf der ersten Platine 121 angeordnet ist) und elektronischen Kanälen der Sendeschaltung 150 und/oder der Empfangsschaltung 160 zu steuern.

Die Mehrzahl an Platinen 120 kann beispielsweise eine siebte Platine 126 mit einem Datenspeicher 185 umfassen. Der Datenspeicher 185 ist ausgebildet, das jeweilige Messsignal des zumindest einen Ultraschallwandlers 140 nach der Verarbeitung durch die Empfangsschaltung 160 zu speichern. Der Datenspeicher 185 kann zur Ablage von Messdaten genutzt werden. Der Datenspeicher 185 kann entweder auf der siebten Platine 126 fest intergiert oder auch austauschbar gestaltet sein. Der Datenspeicher 185 kann sowohl flüchtigen Speicher (z.B. Random Access Memory, RAM) als auch persistenten Speicher wie beispielsweise eine Secure Digital (SD)- Karte, eine Micro-SD-Karte, ein Flash-Speicher, ein Solid-State-Drive (SSD), eine Multimedia Card (MMC), einen per USB-Schnittstelle angebundenen Speicher (z.B. USB-Stick) oder eine Festplatte (engl. Hard Disk Drive, HDD) umfassen. Die siebte Platine kann als ein Datenspeicher-Modul aufgefasst werden. Optional kann der Datenspeicher 185 auch zusammen mit der Prozessierschaltung 180 auf einer gemeinsamen Platine angeordnet sein.

Ebenso kann die Mehrzahl an Platinen 120 ferner eine achte Platine (nicht dargestellt in Figs. 1 und 2) mit einer Positionserfassungsschaltung umfassen. Die Positionserfassungsschaltung ist ausgebildet, eine absolute Position des modularen Ultraschallgeräts 100 und/oder eine relative Position des modularen Ultraschallgeräts in Bezug auf ein vorbestimmtes Objekt zu bestimmen. Je nach Anforderung kann diese Platine die Position und ggf. Lage des modularen Ultraschallgeräts 100 absolut im kleinen oder globalen Maßstab oder relativ z.B. zueinander in einem Sensornetzwerk bestimmen. Die absolute, eigene Positionserfassung kann beispielsweise über einen Empfänger für ein globales Navigationssatellitensystem wie etwa NAVSTAR GPS oder Galileo, die Lokalisierung anhand lokaler drahtloser Netzwerke (engl. Wireless Local Area Network, WLAN) als auch über lokale Positionserfassungssysteme (z.B. Beschleunigungs- und oder Lagesensoren, d.h. Inertialsensoren, optische, akustische oder auch andere Sensoren) erfolgen. Die relative Positionierung des modularen Ultraschallgeräts 100 in einem Sensornetzwerk oder bei verbundenen Einzelgeräten kann z.B. mittels fester mechanischer Schnittstellen, mittels mechanischer Sensorik wie Sensorstreifen (Dehnung, Biegung, ...), mittels externer Positions-Sensorik (z.B. optisch per Bildverarbeitung, optisch per Tracking wie Infrarottracking), mittels Funkreferenzstellen (z.B. Bluetooth Beacons) oder durch die Ultraschallsensorik selbst (z.B. Triangulierung mittels Ultraschall-Signalen im Luftschall oder Immersionsschall je nach Umgebungsmedium) erfolgen. Die achte Platine kann als ein Positionserfassungs-Modul aufgefasst werden.

Optional kann das modulare Ultraschallgerät 100 ferner eine in das Gehäuse integrierte Benutzerschnittstelle (nicht dargestellt) umfassen. Die Benutzerschnittstelle kann beispielsweise ausgebildet sein, optisch und/oder akustisch das modulare Ultraschallgerät 100 betreffende Informationen an einen Benutzer auszugeben. Alternativ oder ergänzend kann die Benutzerschnittstelle ausgebildet sein, eine Benutzereingabe des Benutzers zu empfangen. Die Benutzerschnittstelle kann als ein Anzeige- und Interaktions-Modul aufgefasst werden. Die Benutzerschnittstelle kann beispielsweise zur Anzeige von Status- oder Messwerten und Benutzerinteraktion genutzt werden. Die Benutzerschnittstelle kann z.B. ein Display umfassen, um einfache numerische Werteausgabe zu machen, oder auch einen Bildschirm zur grafischen Ausgabe eines Ultraschallbildes oder eines Kurvenverlaufs. Eine Variante kann auch als Touchscreen ausgelegt sein, um Benutzereingaben entgegenzunehmen. Ebenso kann die Benutzerschnittstelle andere mechanische Eingabemöglichkeiten wie Knöpfe, Taster, Tasten etc. umfassen, um Benutzereingaben zu ermöglichen. Alternativ oder ergänzend können ein oder mehrere Lautsprecher für die Informationsausgabe als auch eines oder mehrere Mikrofone für die Entgegennahme von Benutzereingaben von der Benutzerschnittstelle umfasst sein.

Gemäß Ausführungsbeispielen kann die Mehrzahl an Steckverbinder 131, 132 ausgebildet sein, das jeweilige Stromversorgungssignal von der zweiten Platine 122 zu der jeweiligen weiteren Platine der Mehrzahl an Platinen 120 zu leiten. Mit anderen Worten: Die allgemeine Stromversorgung der einzelnen Platinen kann über die Mehrzahl an Steckverbinder 131, 132 erfolgen. Alternativ kann das jeweilige Stromversorgungssignal von der zweiten Platine 122 zu einer oder mehreren (z.B. zu allen) der Mehrzahl an Platinen 120 über eine andere Schnittstelle erfolgen. Beispielsweise kann sowohl die zweite Platine 122 als auch eine oder mehrere (z.B. alle) der Mehrzahl an Platinen 120 jeweils eine oder mehrere Spulen umfassen, um das jeweilige Stromversorgungssignal induktiv von der zweiten Platine 122 zu der jeweiligen weiteren Platine der Mehrzahl an Platinen 120 zu leiten.

In ähnlicher Weise kann die Mehrzahl an Steckverbinder Signalpfade für den Austausch von Daten zwischen zumindest einem Teil der Mehrzahl an Platinen umfassen. Mit anderen Worten: Die Kommunikation zwischen den einzelnen Platinen kann über die Mehrzahl an Steckverbinder 131, 132 erfolgen. Alternativ kann die Kommunikation zwischen einzelnen Platinen der Mehrzahl an Platinen 120 auch über eine andere Schnittstelle erfolgen. Beispielsweise können einzelne Platinen der Mehrzahl an Platinen 120 Sender und/oder Empfänger für eine entsprechende Drahtloskommunikation umfassen. Beispielsweise kann hierfür Bluetooth, Nahfeldkommunikation (engl. Near-Field Communication, NFC), WLAN, optische Kommunikation oder Kommunikation mittels Schallwellen genutzt werden. Unabhängig von der konkreten Art des Datenaustauschs können die einzelnen Komponenten auf der Mehrzahl an Platinen über einen Kommunikationsbus Daten miteinander austauschen.

Die einzelnen Module bzw. Platinen sind - wie oben ausgeführt - über die Steckverbinder 131, 132 oder andere Schnittstellen (z.B. induktiv, Bluetooth oder andere) über ein entsprechendes Bus-System miteinander verbunden. Es müssen dabei nicht zwangsläufig alle der vorangehend und nachfolgend beschriebenen Module bzw. Platinen vorhanden sein, insbesondere müssen diese nicht in einer bestimmten Reihung angeordnet sein. In bevorzugten Ausführungsformen beinhalten die Steckverbinder 131, 132 sowohl die allgemeine Stromversorgung als auch Daten- und Kommunikations-Bus-Systeme für die funktionale Kommunikation. Auf den Bussen kann sowohl seriell als auch parallele Kommunikation stattfinden.

Für die Kommunikation mit externen Geräten umfasst die Mehrzahl an Platinen 120 ferner eine fünfte Platine (nicht dargestellt in Figs. 1 und 2) mit einer Sendeempfängerschaltung.

Die Sendeempfängerschaltung ist eingerichtet, basierend auf zu übertragenden Daten des mobilen Ultraschallgeräts ein Sendesignal zu erzeugen und entsprechend basierend auf einem Empfangssignal Empfangsdaten für das mobile Ultraschallgerät zu bestimmen.

Die Ausgabe des Sendesignals kann auf vielfältige Weise erfolgen. Beispielsweise kann die Sendeempfängerschaltung ausgebildet sein, das Sendesignal an eine Antenne (nicht dargestellt in Figs. 1 und 2) des modularen Ultraschallgeräts 100 zur Abstrahlung in eine Umgebung des modularen Ultraschallgeräts 100 anzulegen. Die Antenne kann z.B. in das Gehäuse 110 integriert sein oder auf der fünften Platine oder einer separaten Platine angeordnet sein. Alternativ oder ergänzend kann die Sendeempfängerschaltung ausgebildet sein, das Sendesignal an eine Schnittstelle (nicht dargestellt in Figs. 1 und 2) des mobilen Ultraschallgeräts zur drahtgebundenen Kommunikation mit einem externen Gerät anzulegen. Ferner alternativ oder ergänzend kann die Sendeempfängerschaltung ausgebildet sein, das Sendesignal an die Sendeschaltung 150 weiterzuleiten, um das jeweilige Steuerungssignal für den zumindest einen Ultraschallwandler 140 basierend auf dem Sendesignal zu erzeugen und so die zu übertragenden Daten des mobilen Ultraschallgeräts 100 in die von dem zumindest einen Ultraschallwandler 140 ausgesendeten Ultraschallwellen 101 zu kodieren. In analoger Weise kann das Empfangen des Empfangssignals auf vielfältige Weise erfolgen. Beispielsweise kann die Sendeempfängerschaltung ausgebildet sein, das Empfangssignal von der Antenne zu empfangen. Alternativ oder ergänzend kann die Sendeempfängerschaltung ausgebildet sein, das Empfangssignal von der der Schnittstelle zur drahtgebundenen Kommunikation mit dem externen Gerät zu empfangen. Ferner alternativ oder ergänzend kann die Sendeempfängerschaltung ausgebildet sein, das Empfangssignal von der Empfangsschaltung 160 zu empfangen, wobei die Empfangsschaltung 160 eingerichtet ist, das Empfangssignal aus dem jeweiligen Messsignal des zumindest einen Ultraschallwandlers 140 abzuleiten.

Die fünfte Platine kann als ein Kommunikations-Modul aufgefasst werden. Zur Kommunikation und zum Datentransfer zwischen dem modularen Ultraschallgeräte 100 und externer Hardware wie etwa einem mobilen Endgerät oder einem weiteren modularen Ultraschallgerät kann beispielsweise ein standardisiertes Kommunikationsprotokoll genutzt werden. Derart kann das modulare Ultraschallgerät 100 auf einfache Weise mit jedem beliebigen Endgerät, Back-end, etc. je nach Anforderung verbunden werden.

Wie die obigen Ausführungen gezeigt haben, können sowohl kabelgebundene (z.B. drahtgebundene oder faseroptische) als auch kabellose (drahtlose) Schnittstellen genutzt werden. Dabei können grundsätzlich sowohl proprietäre Schnittstellen bzw. Kommunikationsprotokolle als auch standardisierte Schnittstellen bzw. Kommunikationsprotokolle genutzt werden. Für die kabelgebundene Kommunikation können z.B. einfache serielle Schnittstelle wie RS-232 nativ oder seriell über USB, die native USB-Schnittstelle (z.B. USB 2.0 / 3.X / 4), spezielle industrielle Bus-Systeme, herstellerspezifische Schnittstelle (z.B. USB über Lightning), LAN, CAN-Bus oder Mil-Bus genutzt werden. Für die drahtlose Kommunikation können z.B. WLAN, Bluetooth, einfache RF-Funk-Schnittstellen (z.B. 433 MHz / 866 MHz Funk), ZigBee (Z-Wave), LoRaWAN sowie Mobilfunkstandards wie 2G, 4G und 5G oder zukünftige Standards genutzt werden.

Oben wurde bereits angedeutet, dass das modulare Ultraschallgerät 100 mehrere Kommunikationsschnittstellen aufweisen kann. Derart kann das modulare Ultraschallgerät 100 (z.B. die Prozessierschaltung 180 oder die Sendeempfängerschaltung der fünften Platine) dynamisch entscheiden, welche Schnittstelle bzw. welche Schnittstellen genutzt werden. Bei vorhandener Gegenstelle in der Nähe kann z.B. eine Schnittstelle mit hoher Bandbreite genutzt werden. Ohne diese kann auf eine Weitstreckenfunkschnittstelle mit ggf. niedrigerer Bandbreite zurückgegriffen werden. Dies ermöglicht z.B. den automatischen Wechsel zwischen autonomen Betrieb mit Fernübertragung und lokalem Service oder lokalen Auslesen eines Technikers vor Ort. Mit mehreren integrierten Kommunikationsschnittstellen kann das modulare Ultraschallgerät 100 auch automatisch erkennen, ob ein Kommunikationskanal defekt oder gestört ist und folglich einen anderen verfügbaren Kanal / System nutzen.

Wie oben bereits angedeutet, kann das modulare Ultraschallgerät 100 neben einem dedizierten Kommunikationssystem gemäß einigen Ausführungsbeispielen auch mittels Schalls oder Ultraschalls selbst kommunizieren. Bei Nutzung von Ultraschall durch z.B. mehrere modulare Ultraschallgeräte in einem gemeinsamen Medium (z.B. gleicher Raum bei Nutzung von Luftschall oder gleiche Flüssigkeit bei eingetauchter Nutzung) kann neben der Messaufgabe die Erzeugung und Detektion von Ultraschall auch genutzt werden, um neben der eigentlichen Messaufgabe auch Informationen (z. B. Messparameter oder Ergebnisse) zwischen den modularen Ultraschallgeräten selbst auszutauschen.

Die Formatierung der digitalen Kommunikation kann gemäß Ausführungsbeispielen Schnittstellenformate zur Datenübertragung wie beispielsweise JSON, XML oder REST-Service neben klassischen binärformatierten seriellen Übertragungen nutzen.

Im Ausführungsbeispiel der Figs. 1 und 2 sind die Mehrzahl an Platinen 120 übereinander angeordnet und aufeinanderfolgende Platinen der Mehrzahl an Platinen 120 entsprechend mittels der Steckverbinder 131, 132 jeweils zusammengesteckt. Derart können die Mehrzahl an Platinen 120 in Form eines Stapels kompakt angeordnet werden. Es ist jedoch zu beachten, dass die Mehrzahl an Platinen 120 nicht zwangsläufig übereinander angeordnet sein müssen. In alternativen Ausführungsbeispielen können die Mehrzahl an Platinen nebeneinander bzw. lateral zueinander versetzt angeordnet sein. Beispielsweise können die weiteren Platinen der Mehrzahl an Platinen 120 mittels der Steckverbinder 131, 132 auf die erste Platine 121 gesteckt sein. Alternativ können die Mehrzahl an Platinen 120 mittels der Steckverbinder 131, 132 auch auf eine Hauptplatine gesteckt sein. Ebenso können die Mehrzahl an Platinen 120 so angeordnet sein, dass sie einander nur teilweise überdecken. Die Form des Gehäuses 110 kann entsprechend angepasst sein. Hierfür können z.B. rotationssymmetrische Steckverbinder genutzt oder es wird eine Erkennung der Rotation durch Detektion einer Pin-Belegung am Steckverbinder durchgeführt.

Das Gehäuse 110 kann ein modulares Gehäuse sein, welches je nach Anwendung mit kostengünstigen Hüllen (z.B. aus Spritzguss-Kunststoff oder 3D-Druck) überzogen werden kann. Für die gleichen Innendimensionen der zu umhüllenden Elektronik können bei harscheren Umgebungsbedingungen oder (mobilen) Nutzungsbedingungen aber auch stoßfeste (engl. "rugged"), staub- und/oder wasserdichte Gehäuse gewählt werden. Ebenso kann das Gehäuse 110 für besonders hohe Temperaturen oder Drücken ausgeführt sein, um z.B. für Anwendungen im industriellen Umfeld eingesetzt werden zu können. Gehäusevarianten aus Metall können zudem genutzt werden, um eine Wärmeableitung der Elektronikkomponenten im Inneren des Gehäuses 110 zu erleichtern, indem z.B. individuell angepasste Wärmeleitkonstruktionen (z.B. "Heatpipes") von besonders hitzeerzeugenden Modulen bzw. Platinen selbst an das Gehäuse 110 integriert werden.

Optional kann in das Gehäuse 110 ein Befestigungselement (nicht dargestellt in Figs. 1 und 2) zur Befestigung des modularen Ultraschallgeräts 100 an einem Prüfobjekt, d.h. einem mittels des modularen Ultraschallgeräts 100 zu prüfenden Objekts, integriert ist. Das Befestigungselement kann auf vielfältige Weise ausgeführt sein. Das Befestigungselement kann z.B. ein Rahmen zur Aufnahme von Klebepads sein, um das modulare Ultraschallgerät 100 an das Prüfobjekt zu kleben. Alternativ kann das Befestigungselement z.B. ein Rahmen mit Ösen für einen oder mehrere Riemen, eine oder mehrere Schlauchschellen oder eine Halterung mit Ösen für Kabelbinder oder ähnliches sein.

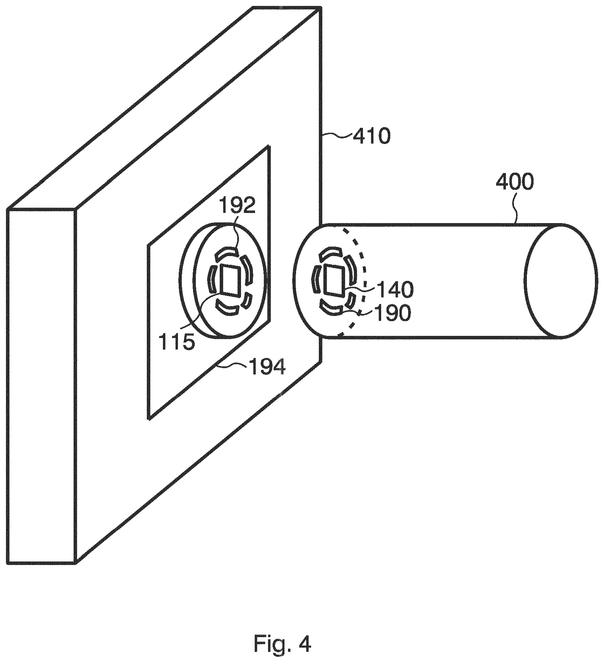

Nachfolgend werden unter Bezugnahme auf Fig. 3 und Fig. 4 zwei mögliche Ausgestaltungen des Befestigungselements näher erläutert.

**Fig. 3** zeigt ein weiteres modulares Ultraschallgerät 300, das gemäß den vorangehenden Ausführungen ausgeführt ist. Wie aus einem Vergleich von Fig. 1 und Fig. 3 ersichtlich ist, weist das Gehäuse des modularen Ultraschallgeräts 300 im Gegensatz zum Gehäuse des modularen Ultraschallgeräts 100 keine Würfelform auf, sondern ist länglich - ähnlich einem Stab - ausgeführt.

Weiter ist in Fig. 3 ein magnetisches Prüfobjekt (Prüfling) 310 wie etwa eine metallische (Wand-)Fläche, ein Rohr oder eine andere metallische Oberfläche gezeigt. Die Positionierung des modularen Ultraschallgeräts 300 an dem Prüfobjekt 310 erfolgt über eine Magnethalterung im vorderen Bereich des modularen Ultraschallgeräts 300. Das Befestigungselement des modularen Ultraschallgeräts 300 umfasst dazu einen oder mehrere Magnete 190, die so angeordnet sind, dass sie den zumindest einen Ultraschallwandler 140 des modularen Ultraschallgeräts 300 nicht behindern. Beispielsweise können der eine oder die mehreren Magnete 190 in das Gehäuse des modularen Ultraschallgeräts 300 oder die erste Platine, welche den zumindest einen Ultraschallwandler 140 trägt, integriert sein. Die vorgeschlagene Magnethalterung ermöglicht eine direkte Anbindung/Positionierung des modularen Ultraschallgeräts 300 an einem z.B. metallischen Prüfling. Gegebenenfalls kann zusätzlich ein trockenes Koppelpad oder ein Koppelmedium zwischen der Magnethalterung und dem Prüfobjekt genutzt werden.

Obwohl in Fig. 3 eine Befestigung des modularen Ultraschallgeräts 300 auf einer im Wesentlichen planen Fläche des Prüfobjekts 310 gezeigt ist, ist zu beachten, dass sowohl der vordere Bereich des modularen Ultraschallgeräts 300 als auch das Gegenstück in Form des Prüfobjekts jegliche beliebige Form aufweisen können. Beispielsweise können das Prüfobjekt 310 auch ringförmig gebildet sein und der vordere Bereich des modularen Ultraschallgeräts 300 entsprechend angepasst sein.

Ebenso ist zu beachten, dass die Magnethalterung für bestimmte Anwendungen in analoger Weise auch im hinteren Bereich bzw. einer Rückseite des modularen Ultraschallgeräts 300 angebracht sein kann. Dies kann eine einfache Montage z.B. für Luftschallanwendungen an metallischen Oberflächen ermöglichen.

**Fig. 4** zeigt im Weiteren eine alternative Ausführung des Befestigungselements für ein nicht-magnetisches Prüfobjekt 410. Im Beispiel der Fig. 4 wird ein Magnet-Klebe-Adapter als Befestigungselement genutzt, um das modulare Ultraschallgerät 300 an dem nichtmagnetischen Prüfobjekt 410 zu befestigen.

Das Befestigungselement des modularen Ultraschallgeräts 400 besteht dazu aus zwei miteinander verbindbaren bzw. trennbaren Teilen. Der erste Teil des Befestigungselements ist durch einen oder mehrere Magnete 190 gebildet, die so angeordnet sind, dass sie den zumindest einen Ultraschallwandler 140 des modularen Ultraschallgeräts 300 nicht behindern. Beispielsweise können der eine oder die mehreren Magnete 190 in das Gehäuse des modularen Ultraschallgeräts 400 oder die erste Platine, welche den zumindest einen Ultraschallwandler 140 trägt, integriert sein. Der zweite Teil des Befestigungselements ist durch eine Klebefläche oder andersartig mechanisch zu befestigende Fläche 194, auf dem einer oder mehrere Gegenmagnete 194 ausgebildet bzw. angeordnet sind, welche mit dem einen oder den mehreren Magneten 190 magnetisch koppeln können, um das modularen Ultraschallgerät 400 an der Klebefläche 194 und mittels Anklebens der Klebefläche 194 an dem Prüfobjekt 410 an dem Prüfobjekt 410 zu befestigen. Wie in Fig. 4 dargestellt, können der eine oder die mehreren Gegenmagnete 194 in einem Gehäuseteil gehalten sein, das bündig mit dem übrigen Gehäuse des modularen Ultraschallgeräts 400 abschließen kann, um eine optimale Positionierung des modularen Ultraschallgeräts 400 an dem Prüfobjekt 410 zu ermöglichen.

Im Beispiel der Fig. 4 wird somit ein Gegenstück zur reproduzierbaren Positionierung des modularen Ultraschallgeräts 400 genutzt, das selbst magnetisch ist und mittels Adapter (z.B. adhäsiver Fläche) auf das Prüfobjekt 410 aufgebracht wird. Der Magnet-Klebe-Adapter kann genau passend durch Integration einzelner Magnete in einer eindeutigen Positionierung sein, die eine Kodierung der Magnetpositionen bieten.

Die magnetische Befestigung sowohl an der Wandlerfront des modularen Ultraschallgeräts seitens eines Prüfobjekts als auch rückseitig zur Montage des modularen Ultraschallgeräts kann ggf. durch elektromagnetische Funktion im Betrieb verstärkt werden. Auch hier kann ggf. ein Gegenstück notwendig sein und genutzt werden, wenn das Prüfobjekt, an dem das modulare Ultraschallgerät befestigt werden soll, keine metallische und magnetische Oberfläche besitzt.

Die Hardware der vorangehend und nachfolgend beschriebenen modularen Ultraschallgeräte stellt ein variables, steckbares Baukastensystem dar, das aus aufeinander abgestimmten Einzelmodulen bzw. Platinen besteht.

Das jeweilige Gehäuse der vorangehend und nachfolgend beschriebenen modularen Ultraschallgeräte erstreckt sich unabhängig von seiner konkreten Form dreidimensional im Raum, d.h. in drei zueinander senkrechten Raumrichtungen. Die hierin vorgeschlagenen modularen Ultraschallgeräte können sehr kompakt ausgeführt werden, so dass sie z.B. bequem in einer menschlichen Hand aufgenommen und von dieser über ein Prüfobjekt bewegt werden können. Beispielsweise kann eine Erstreckung des Gehäuses in jeder der drei zueinander senkrechten Raumrichtungen weniger als 15 cm, 10 cm oder 5 cm betragen.

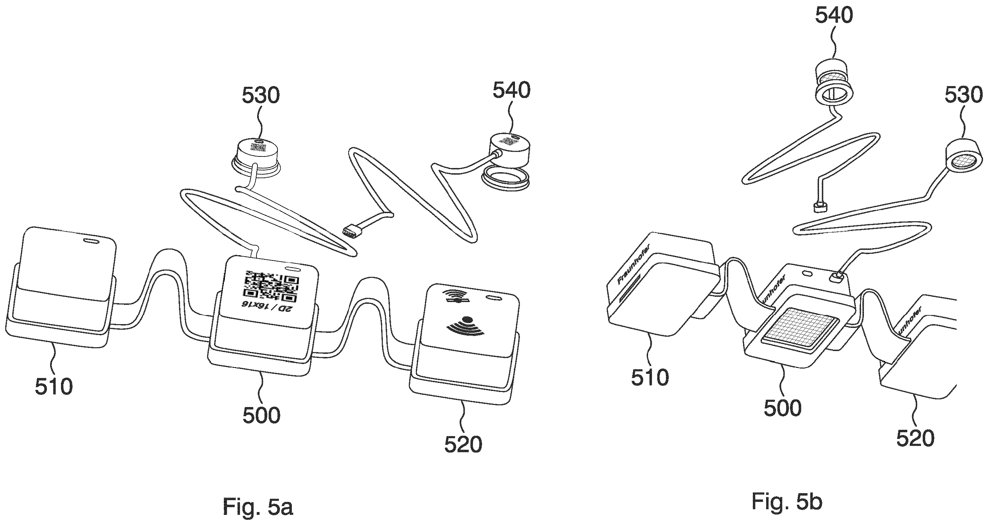

**Fig. 5a** und **Fig. 5b** zeigen perspektivisch ein weiteres modulares Ultraschallgerät 500, das mit Zusatzgeräten 510, 520, 530 und 540 gekoppelt ist.

Das modulare Ultraschallgerät 500 ist dabei gemäß der vorbeschriebenen Grundkonfiguration ausgeführt. Mit anderen Worten: Das modulare Ultraschallgerät 500 umfasst neben dem zumindest einen Ultraschallwandler noch die Sendeschaltung, die Empfangsschaltung und die Stromversorgungsschaltung. Im Zusatzgerät 510, mit dem modulare Ultraschallgerät 500 per Kabel gekoppelt ist, ist ein Akkumulator vorgesehen, welcher die elektrische Energie für die Stromversorgungsschaltung bereitstellt. Im Zusatzgerät 520, mit dem modulare Ultraschallgerät 500 per Kabel gekoppelt ist, ist die oben beschriebene Sendeempfängerschaltung vorgesehen, so dass das modulare Ultraschallgerät 500 über das Zusatzgerät 520 mit externen Geräten kommunizieren kann. Zusätzlich ist das modulare Ultraschallgerät 500 per Kabel mit zwei weiteren Zusatzgeräten 530 und 540, die weitere Ultraschallwandler aufweisen, verbunden. Die Sendeschaltung und die Empfangsschaltung können über die Kabel jeweilige Steuerungssignal an die weiteren Ultraschallwandler der Zusatzgeräte 530 und 540 ausgeben und entsprechend Messsignale der weiteren Ultraschallwandler der Zusatzgeräte 530 und 540 empfangen.

Mittels der Zusatzgeräte 510, 520, 530 und 540 kann die Funktionalität des modularen Ultraschallgerät 500 erweitert werden.

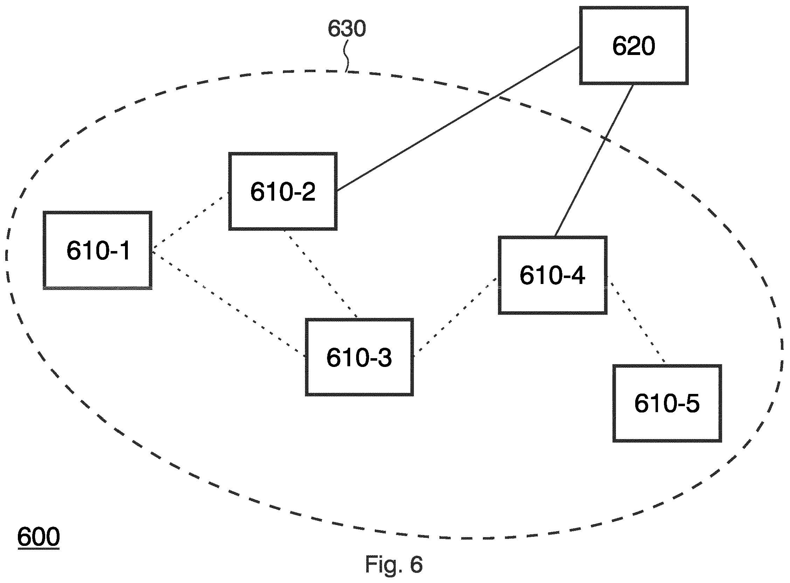

Nachdem vorangehend die Einzelheiten verschiedener modularer Ultraschallgeräte gemäß der vorliegenden Offenbarung beschrieben wurden, werden nachfolgend einige Ultraschallsysteme, welche modulare Ultraschallgeräte gemäß der vorliegenden Offenbarung nutzen, unter Bezugnahme auf die Figs. 6 bis 9 näher beschrieben.

**Fig. 6** zeigt ein Ultraschallsystem 600, welches neben einer Vorrichtung 620 zur Auswertung von Messdaten noch fünf modulare Ultraschallgeräte 610-1, ..., 610-5 gemäß der vorliegenden Offenbarung aufweist. Obwohl im Ausführungsbeispiel der Fig. 6 genau fünf modulare Ultraschallgeräte gezeigt sind, versteht sich von selbst, dass die vorliegende Offenbarung nicht darauf beschränkt ist und ein Ultraschallsystem gemäß der vorliegenden Offenbarung grundsätzlich jede beliebige Mehrzahl an modularen Ultraschallgeräten gemäß der vorliegenden Offenbarung umfassen kann.

Die modularen Ultraschallgeräte 610-1, ..., 610-5 sind dabei jeweils zumindest gemäß der vorbeschriebenen Grundkonfiguration ausgeführt und umfassen jeweils zusätzlich die oben beschriebene fünfte Platine mit der Sendeempfängerschaltung auf. Die modularen Ultraschallgeräte 610-1, ..., 610-5 bilden ein Sensornetzwerk bzw. Mesh 630.

Die modularen Ultraschallgeräte 610-1, ..., 610-5 sind ausgebildet, jeweilige Messdaten direkt oder durch Weiterleitung über zumindest ein weiteres der modularen Ultraschallgeräte 610-1, ..., 610-5 zu einem oder mehreren vorbestimmten modularen Ultraschallgeräten der Ultraschallgeräte 610-1, ..., 610-5 zu senden. Im Beispiel der Fig. 6 sind die modularen Ultraschallgeräte 610-2 und 610-4 vorbestimmte Ultraschallgeräte. Wie aus Fig. 6 ersichtlich ist, sendet das modulare Ultraschallgerät 610-1 seine Messdaten unmittelbar an das vorbestimmte Ultraschallgerät 610-2 und über das Ultraschallgerät 610-3, welches als Relaisstation dient, an das vorbestimmte Ultraschallgerät 610-4. Das modulare Ultraschallgerät 610-3 sendet seine Messdaten unmittelbar an die vorbestimmten modularen Ultraschallgeräte 610-2 und 610-4. Das modulare Ultraschallgerät 610-3 sendet seine Messdaten unmittelbar an das vorbestimmte Ultraschallgerät 610-4.

Die vorbestimmten modularen Ultraschallgeräte 610-2 und 610-4 sind ausgebildet, die gesammelten Messdaten der modularen Ultraschallgeräten 610-1, ..., 610-5 an die Vorrichtung 620 zur Auswertung von Messdaten zu senden. Die Vorrichtung 620 zur Auswertung von Messdaten ist wiederum ausgebildet, eine oder mehrere vorbestimmte Kenngrößen basierend auf den empfangenen Messdaten zu bestimmen. Die Bestimmung der einen oder mehreren vorbestimmte Kenngrößen kann wie vorangehend beschrieben erfolgen. Beispielsweise kann die Vorrichtung zur Auswertung von Messdaten ausgebildet sein, die eine oder die mehreren vorbestimmten Kenngrößen unter Verwendung eines mittels maschinellen Lernens trainierten Modells aus den Messdaten zu bestimmen. Für die Bestimmung der einen oder mehreren vorbestimmte Kenngrößen kann die Vorrichtung 620 zur Auswertung von Messdaten eine entsprechende Prozessierschaltung wie etwa einen Prozessor, eine CPU, einen ASIC, einen IC, einen SoC, einen FPGA mit einem Mikroprozessor oder einem komplexeren lokalen Rechencluster aufweisen, auf dem/der Software für die Bestimmung der einen oder mehreren vorbestimmte Kenngrößen gemäß den hierin beschriebenen Grundsätzen abläuft. Ferner kann die Prozessierschaltung der Vorrichtung 620 zur Auswertung von Messdaten einen oder mehrere Speicher aufweisen bzw. mit diesen gekoppelt sein.

Wie durch die gestrichelten Linien zwischen den modularen Ultraschallgeräten 610-1, ..., 610-5 in Fig. 6 angedeutet, können die modularen Ultraschallgeräten 610-1, ..., 610-5 z.B. ausgebildet sein, die Messdaten drahtlos mittels Kurzstreckenfunk zu den vorbestimmten modularen Ultraschallgeräte 610-2 und 610-4 zu senden. Dies kann eine energiesparende Datenübertragung ermöglichen. Wie durch die Wellenlinien zwischen den modularen Ultraschallgeräte 610-2 und 610-4 und der Vorrichtung 620 zur Auswertung von Messdaten in Fig. 6 angedeutet, können die modularen Ultraschallgeräte 610-2 und 610-4 können entsprechend ausgebildet sein, die gesammelten Messdaten der modularen Ultraschallgeräten 610-1, ..., 610-5 mittels Langstreckenfunk an die Vorrichtung 620 zur Auswertung von Messdaten zu senden.

Neben der Kommunikation zu einer zentralen Instanz können die modularen Ultraschallgeräten 610-1, ..., 610-5 somit die Bildung eines Sensornetzwerks bzw. Meshs 630 ermöglichen, um Messdaten über weitere Strecken trotz eingesetztem Kurzstreckenfunks zu übertragen. Das Einfügen von einzelnen modularen Ultraschallgeräten bzw. Knoten mit Langstreckenfunkfunkionalität in das Sensornetzwerk kann helfen die Fehleranfälligkeit zu reduzieren (z.B. Ausfall eines Knotens führe sonst zu Unterbrechung der Kommunikation).

Die modularen Ultraschallgeräte 610-1, ..., 610-5 können alternativ auch kabelgebunden miteinander Daten austauschen. Ebenso kann der Datenaustausch mit der Vorrichtung 620 zur Auswertung von Messdaten bei Bedarf kabelgebunden erfolgen.

Ein Kommunikationskanal kann auch zur Fehlerkommunikation im lokalen Sensornetzwerk bzw. Mesh 630 zu anderen modularen Ultraschallgeräten genutzt werden. So können Statusmeldungen in dem Sensornetzwerk 630 übertragen werden. Auch im Normalbetrieb kann eine Messwertkommunikation im lokalen Sensornetzwerk bzw. Mesh 630 zu anderen Knoten bzw. modularen Ultraschallgeräten durchgeführt werden. So können Messwerte oder Klassifikationen wie beispielsweise "I-O" ("in Ordnung") Status oder "N-I-O" ("nicht in Ordnung") Status von Prüfobjekten übertragen werden. Dies kann bei weiteren modularen Ultraschallgeräten im Sensornetzwerk bzw. Mesh 630 dann ggf. andere und angepasste Messungen des gleichen Prüfobjektes in einer Prüfsequenz auslösen.

Weiterhin können die modularen Ultraschallgeräte 610-1, ..., 610-5 zum Beispiel auch für Langzeitmessaufgaben zur Zustandsüberwachung (z. B. in der Pflege oder in der Bauwerksüberwachung usw.) autark betrieben werden und in festgelegten Intervallen Daten erheben. Der Datentransfer kann mittels des Kommunikationsmoduls z.B. gemäß eines Mobilfunkstandards erfolgen.

Zwei oder mehrere der modularen Ultraschallgeräte 610-1, ..., 610-5 können in einen synchronisierten Betrieb gesetzt werden und beispielsweise einer nur als Sender und einer als Empfänger arbeitet.

Wenn zumindest einige der modularen Ultraschallgeräte 610-1, ..., 610-5 bei Langzeitmessung mit einem Datenspeicher gemäß den obigen Ausführungen versehen sind, ist es möglich, die gespeicherten Daten im aktiv in bestimmten Zeitabständen zu erfassen (z.B. beim Passieren bzw. im Vorbeigehen).

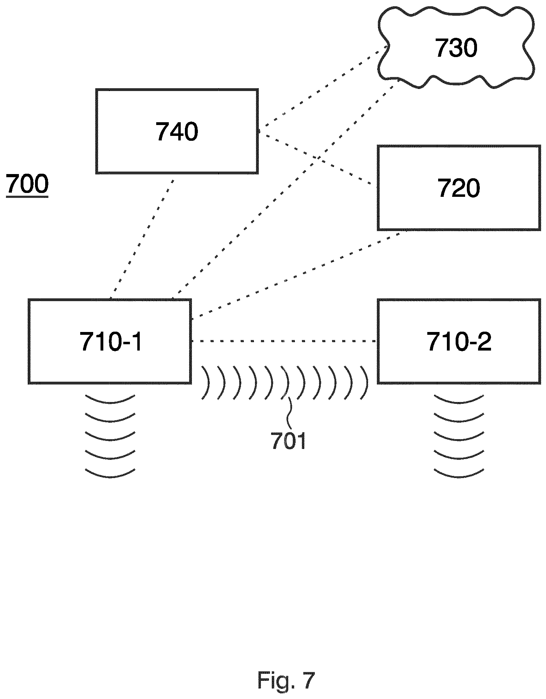

**Fig. 7** zeigt ein weiteres Ultraschallsystem 700 mit zwei modularen Ultraschallgeräten 710-1 und 710-2. Wie durch die gestrichelte Linie zwischen den modularen Ultraschallgeräten 710-1 und 710-2 in Fig. 7 angedeutet, sind die modularen Ultraschallgeräten 710-1 und 710-2 analog zu dem oben beschriebenen ausgebildet, die Messdaten drahtlos mittels Kurzstreckenfunk auszutauschen. Im Gegensatz zum Ultraschallsystem 600 sind die modularen Ultraschallgeräte 710-1 und 710-2 zusätzlich ausgebildet, die Messdaten durch Kodierung in die ausgesendeten Ultraschallwellen 701 auszutauschen. Die Kodierung der ausgesendeten Ultraschallwellen 701 erfolgt dabei gemäß den oben beschriebenen Grundsätzen. Den modularen Ultraschallgeräte 710-1 und 710-2 steht somit ein weiterer Kommunikationskanal zur Verfügung.

Im Beispiel der Fig. 7 ist das modulare Ultraschallgerät 710-1 das vorbestimmte Ultraschallgerät des Ultraschallsystems 700 und leitet die gesammelten Messdaten der modularen Ultraschallgeräte 710-1 und 710-2 zur Auswertung weiter. Wie in Fig. 7 angedeutet kann das vorbestimmte Ultraschallgerät 710-1 die gesammelten Messdaten nicht nur an eine Vorrichtung zur Auswertung von Messdaten weiterleiten, wie dies im Ausführungsbeispiel der Fig. 6 der Fall ist, sondern auch an mehrere Vorrichtungen 720, 730 und 740 zur Auswertung von Messdaten.

Die einzelnen Vorrichtungen 720, 730 und 740 können unterschiedlich leistungsfähig und für unterschiedliche Anwendungen ausgelegt sein. Beispielsweise kann die Vorrichtung 720 ein Edge-KI-System sein, das mittels KI-basiertem Edge-Computing die gesammelten Messdaten der modularen Ultraschallgeräte 710-1 und 710-2 auswertet. In ähnlicher Weise kann die Vorrichtung 730 beispielsweise ein Cloud-KI-System sein, das in der Cloud eine KI-basierte Auswertung der gesammelten Messdaten der modularen Ultraschallgeräte 710-1 und 710-2 vornimmt. Die Vorrichtung 740 kann beispielsweise ein mobiles Endgerät wie ein Tablet-Computer oder ein Mobiltelefon sein, auf dem Software für die Auswertung und/oder Visualisierung der gesammelten Messdaten der modularen Ultraschallgeräte 710-1 und 710-2 läuft. Die Vorrichtungen 720, 730 und 740 können auch untereinander Daten austauschen und so z.B. einzelne Schritte der Auswertung und/oder Visualisierung der gesammelten Messdaten der modularen Ultraschallgeräte 710-1 und 710-2 auf die einzelnen Vorrichtungen verteilen.

Über ein entsprechendes Softwarekonzept können das vorangehend beschriebene Hardwarekonzept bzw. die verteilten Sensornetzwerke effizient und für spezifische Anwendungen anpassbar gemacht werden. Neben der Software / Firmware auf den jeweiligen modularen Ultraschallgeräten selbst (z.B. Hardwaresteuerung, Schallstrahlsteuerung im Sende- und Empfangspfad, Plug-In basierte Filterung für anwendungsspezifische KI-Signalverarbeitung und Parameterextraktion, Bildgebung und -analyse oder Kommunikation mit Endgeräten) kann die Software z.B. die Kommunikation zwischen mehreren Einzelsystemen und die Kommunikation mit lokaler Edge-KI und entfernter Cloud-KI unterstützen. So können die einzelnen Elemente für die unterschiedlichen Anwendungsszenarien individuell entwickelt, optimiert und eingesetzt werden, um analog zur Hardware auch eine anwendungsspezifische Modularität des Softwarekonzeptes zu ermöglichen.

Über das App-basierte Softwarekonzept, können durch Softwareupdates ebenfalls neue Anwendungsbereiche für die gleiche Hardware erschlossen werden und somit die Flexibilität der vorgeschlagenen modularen Ultraschallgeräte erhöht werden. Durch die komplette Verlagerung von Signalverarbeitung und Bildgebung in die Software können Hardwareressourcen und somit Kosten gespart.

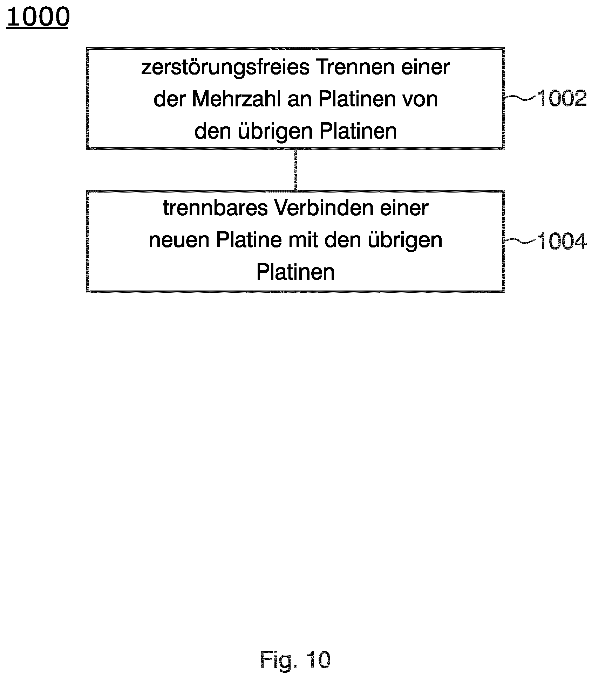

Im Hinblick auf die spätere Weiterentwicklung der vorgeschlagenen Ultraschallsystem ist der modulare Gesamtansatz von erheblichem Vorteil. Zum einen kann die Entwicklungszeit für neue Anwendungsfelder und die damit einhergehende "Time-to-Market"-Zeit erheblich reduziert werden. Zum anderen macht das Baukastenprinzip zusammen mit der Variabilität des Softwarekonzepts (z.B. nachladbare Apps) das System zukunftssicher, flexibel und kosteneffizient in seiner Weiterentwicklung. Wird bei einem der Einzelmodule ein technologischer Innovationssprung erreicht (z.B. Energieversorgung, breitbandigere Funkstandards), muss nicht das gesamte System neu entworfen werden, sondern nur das entsprechende Modul ausgetauscht werden, wie dies später unter Bezugnahme auf Fig. 10 noch näher ausgeführt wird.

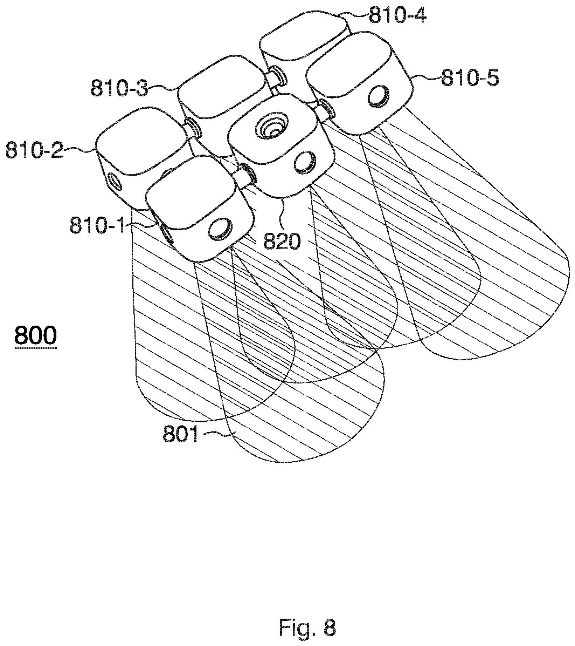

**Fig. 8** zeigt ein weiteres Ultraschallsystem 800 mit fünf modularen Ultraschallgeräten 810-1, ..., 810-5 gemäß der vorliegenden Offenbarung. Analog zu den modularen Ultraschallgeräten der Ausführungsbeispiele der Figs. 6 und 7 sind auch die modularen Ultraschallgeräte 810-1, ..., 810-5 jeweils zumindest gemäß der vorbeschriebenen Grundkonfiguration ausgeführt und umfassen jeweils zusätzlich die oben beschriebene fünfte Platine mit der Sendeempfängerschaltung auf. Im Ausführungsbeispiel der Fig. 8 sind die Gehäuse der fünf modularen Ultraschallgeräte 810-1, ..., 810-5 über entsprechende Kopplungselemente aneinander befestigt, um ein kompaktes Sensornetzwerk zu bilden. Zudem ist ein Zusatzgerät 820 mit den modularen Ultraschallgeräten 810-1, ..., 810-5 gekoppelt. Im Zusatzgerät 820 ist ein Akkumulator vorgesehen, welcher die elektrische Energie für die Stromversorgungsschaltungen der modularen Ultraschallgeräten 810-1, ..., 810-5 bereitstellt.

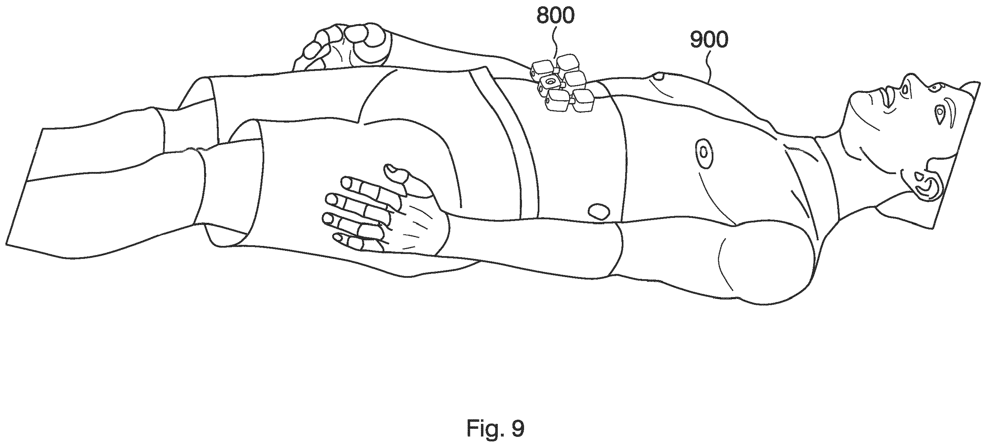

Über die durch die Sendeempfängerschaltungen der modularen Ultraschallgeräten 810-1, ..., 810-5 bereitgestellten Hardware- und Software-Schnittstellen ist eine Kombination und Vernetzung mehrerer modularer Ultraschallgeräte über eine kabellose Schnittstelle zu einem Sensornetzwerk möglich. Mittels der überlappenden Schallwellen 801 der modularen Ultraschallgeräten 810-1, ..., 810-5 kann ein großflächigerer Bereich mittels Ultraschalls untersucht werden. Damit wird durch eine integrierte Bestimmung der Einzelpositionen zueinander und durch das softwarebasierte Vernetzen der einzelnen Messdaten die parallele bzw. gleichzeitige Erfassung von größeren Bereichen durch eine in Fig. 8 nicht dargestellte, separate Vorrichtung zur Auswertung von Messdaten möglich. Dies ist beispielhaft in Fig. 9 dargestellt, wo das Ultraschallsystem 800 für die Ultraschalluntersuchung des Abdomens einer Person 900 genutzt wird.

Wie bereits oben mehrfach angedeutet ermöglichen die modularen Ultraschallgeräte gemäß der vorliegenden Offenbarung den Austausch einzelner Module bzw. Platinen. Um diesen Aspekt der vorliegenden Offenbarung nochmals ausführlicher zu beleuchten, wird nachfolgend unter Bezugnahme auf Fig. 10 ein Verfahren 1000 zur Modifikation eines modularen Ultraschallgeräts gemäß der vorliegenden Offenbarung näher beschrieben. Beispielsweise kann das Verfahren 1000 genutzt werden, um eine der Mehrzahl an Platinen 120 des in Figs. 1 und 2 gezeigten modularen Ultraschallgeräts 100 zu tauschen.

Das Verfahren 1000 umfasst ein zerstörungsfreies Trennen 1002 einer der Mehrzahl an Platinen von den übrigen Platinen der Mehrzahl an Platinen durch Lösen zumindest eines Teils der Steckverbinder. Beispielsweise kann hierzu zunächst das Gehäuse des modularen Ultraschallgeräts zerstörungsfrei geöffnet und die Mehrzahl an Platinen oder zumindest ein Teil davon aus dem Gehäuse entnommen werden. Um bei dem in Figs. 1 und 2 gezeigten modularen Ultraschallgerät 100 z.B. die erste Platine 121 zu tauschen, kann z.B. die Mehrzahl an Platinen 120 aus dem Gehäuse 110 entnommen werden und anschließend die erste Platine 121 durch Lösen der Steckverbinder, welche die erste Platine 121 mit der dritten Platine 123 verbinden, zerstörungsfrei von den übrigen Platinen der Mehrzahl an Platinen 120 getrennt werden.

Ferner umfasst das Verfahren 1000 ein trennbares Verbinden 1004 einer neuen Platine mit den übrigen Platinen der Mehrzahl an Platinen mittels eines oder mehrerer Steckverbinder. Die neue Platine kann beispielsweise die gleiche oder eine ähnliche Funktionalität wie die eine der Mehrzahl an Platinen, die von den übrigen Platinen der Mehrzahl an Platinen durch Lösen zumindest eines Teils der Steckverbinder getrennt wird bzw. wurde, aufweisen. Bezugnehmen auf das obige Beispiel kann beispielsweise eine neue Platine mit einem anderen Ultraschallwandler über die Steckverbinder mit der dritten Platine 123 verbunden werden. Soll das modulare Ultraschallgerät 100 z.B. zunächst für eine erste Anwendung und anschließend für eine zweite Anwendung mit unterschiedlichen Anforderungen an den verwendeten Ultraschall (z.B. Ultraschallgeometrie oder Ultraschallfrequenz) verwendet werden, kann durch den Tausch der Platine mit dem zumindest einen Ultraschallwandler das modulare Ultraschallgerät 100 auf einfache Weise an die jeweilige Anwendung angepasst werden.

Ebenso können gemäß dem Verfahren 1000 z.B. defekte Platinen auf einfache Weise getauscht werden. Da nur die jeweilige Platine getauscht werden muss bei einem Defekt, kann ein modulares Ultraschallgerät gemäß der vorliegenden Offenbarung auf einfach und kostengünstige Weise repariert werden. Auch können Platinen für bestimmte Funktionalitäten gemäß dem Fortschreiten der Technik aktualisiert werden. Beispielsweise kann bei Aufkommen eines neuen Kommunikationsstandards die Platine mit der Sendeempfängerschaltung gemäß dem Verfahren 1000 auf einfache Weise gegen eine modernere Platine mit einer Sendeempfängerschaltung, die den neuen Kommunikationsstandard unterstützt, getauscht werden, so dass ein modulares Ultraschallgerät gemäß der vorliegenden Offenbarung auch mit modernsten Drittgeräten kommunizieren kann.

Gemäß der vorliegenden Offenbarung wird ein Baukasten für Ultraschallanwendungen mit einer modularen Hardwarebasis und verschiedenen Software-Apps (z.B. im System, im mobilen Device, im lokalen Edge-KI-System, in der Cloud) bereitgestellt. Der Baukasten ermöglicht durch applikationsspezifische Kombination die Adressierung eines sehr weiten Anwendungsbereiches. Reicht dieser nicht aus oder kommt es zu großen technologischen Innovationssprüngen (z.B. neue Akku- oder Funk-Technologie) muss nur ein Sub-Modul bzw. eine Platine neu entwickelt werden, so dass das modulare Ultraschallgerät sehr einfach durch Austausch des entsprechenden Moduls bzw. der entsprechenden Platine und je nach Anwendung ggf. durch Anpassung Software angepasst werden kann.

Vorteile des Baukastens sind daher eine hohe Anwendungsbandbreite durch einfache Rekonfiguration als auch große Zukunftssicherheit, da neue Technologien einfach in entsprechende Module bzw. Platinen überführt werden können. Somit ist das Konzept zudem sehr nachhaltig (neue Anwendungen und Technologien bedürfen nicht immer gleich einer kompletten Neuentwicklung und Herstellung) und es werden in großen Umfang Entwicklungskosten verringert. Ein Anwender kann langfristig immer den aktuellen Stand der Technik in allen Submodulen bzw. Platinen nutzen und das System bei Bedarf technologisch um weitere Sensorklassen erweitern (neue Module für z. B.: Druckmessung, Helligkeit, Geruch, Feuchte, Härte, etc.). Zukünftige Technologiesprünge in einzelnen Komponenten können durch Austausch des entsprechenden Moduls direkt ausgenutzt werden, ohne ein Redesign der gesamten Elektronikeinheit durchführen zu müssen.

Mit dem vorgeschlagenen Baukasten ist es perspektivisch einfacher, schneller, nachhaltiger und kostengünstiger möglich, neue Ultraschallsysteme und Verfahren zu entwickeln. Zudem können diese flexibel an eine sehr große Bandbreite von möglichen unterschiedlichen Anwendungsfeldern (z.B. Pflege, Fitness, Industrie 4.0. Medizin, etc.) durch Updates der Software-Module und unterschiedliche Apps bei gleicher Hardwarebasis für die Messtechnik angepasst werden.

Bei einer technologisch breiten Auslegung der Hardware wird allein durch die applikationsspezifischen Software-Module ein sehr weiter Anwendungsbereich abgedeckt. Wenn die Leistung eines einzelnen Hardware-Moduls bzw. einer einzelnen Platine für eine Anwendung nicht ausreicht, können die einzelnen Module bzw. Platinen sehr einfach durch den Baukasten-Ansatz hinsichtlich der jeweiligen Anwendung gewählt und frei rekombiniert werden.

Durch die hohe Flexibilität und Kosteneffizienz des Technologieansatzes ergibt sich eine sehr große Anwendungsbandbreite. Beispielsweise ist die Anwendung im Medizin- bzw. Pflegebereich, im Consumer- bzw. Prosumerbereich oder im Industriebereich möglich.

Die vorliegende Offenbarung gibt eine modulare Ultraschall-Sensor-Elektronik und Software an, die flexibel für verschiedenste Anwendungsfelder konfiguriert werden kann, und dabei so kostengünstig ist, dass im medizinischen als auch technischen Kontext Massenmärkte adressiert werden können.

Die oben beschriebene Vielzahl an unterschiedlichen Modulen bzw. Platinen können in Abhängigkeit der Applikation zu einem Gesamtsystem kombiniert werden. Anforderungen hinsichtlich des anwendungs-spezifischen Frequenzbereichs, der Ausgangsleistung usw. definieren dabei die Anzahl der notwendigen Module bzw. Platinen, welche flexibel über z.B. standardisierte Hard- und Software-Schnittstellen konfiguriert werden. Akkumulatorbasierte Lösungen sind ebenso denkbar wie auch Speichermodule zum Aufzeichnen der Daten von Langzeitmessungen. Mehrere dieser Ultraschallsysteme können auch über eine z.B. kabellose Schnittstelle zu einem intelligenten Sensornetzwerk kombiniert und Ressourcen zwischen den Sensorsystemen geteilt werden. Zur Kommunikation und zum Datentransfer zwischen einem mobilen Endgerät und dem modularen Ultraschallgerät kann z.B. ein Modul mit einer herstellerunabhängigen Funkschnittstelle oder eine Kabelverbindung mit einem standardisierten Kommunikationsprotokoll integriert werden. Somit kann das System einfach mit jedem Endgerät entsprechender Leistung verbunden und nach Laden der entsprechenden applikationsbezogenen Software in Betrieb genommen werden.

Die vorliegende Offenbarung gibt ein autark arbeitendes Gesamtkonzept an, das sehr kostengünstig ist und zudem je nach Notwendigkeit und Applikation, entweder die stetig wachsende Leistungsfähigkeit mobiler Consumer-Endgeräte über Funkschnittstellen (z.B. Bluetooth, WLAN, etc.) einbindet und dabei die »Intelligenz« von der Hardware hin zur Software, die auf diesen Consumer-Geräten läuft, nutzt oder diese mobilen Endgeräte als Brücke zu z.B. KI-Cloud-Anwendungen nutzt oder direkt über die integrierte Hardware eine Anbindung zur Cloud hat und dort KI-Anwendungen ermöglicht. Automatisierte intelligente Auswerteverfahren können so in einem Cloud-Netzwerk oder als Edge-KI mit integrierter Datenverarbeitungskapazität durchgeführt werden.

Die Aspekte und Merkmale, die im Zusammenhang mit einem bestimmten der vorherigen Beispiele beschrieben sind, können auch mit einem oder mehreren der weiteren Beispiele kombiniert werden, um ein identisches oder ähnliches Merkmal dieses weiteren Beispiels zu ersetzen oder um das Merkmal in das weitere Beispiel zusätzlich einzuführen.

Es versteht sich ferner, dass die Offenbarung mehrerer, in der Beschreibung oder den Ansprüchen offenbarter Schritte, Prozesse, Operationen oder Funktionen nicht als zwingend in der beschriebenen Reihenfolge befindlich ausgelegt werden soll, sofern dies nicht im Einzelfall explizit angegeben oder aus technischen Gründen zwingend erforderlich ist. Daher wird durch die vorhergehende Beschreibung die Durchführung von mehreren Schritten oder Funktionen nicht auf eine bestimmte Reihenfolge begrenzt. Ferner kann bei weiteren Beispielen ein einzelner Schritt, eine einzelne Funktion, ein einzelner Prozess oder eine einzelne Operation mehrere Teilschritte, -funktionen, -prozesse oder -operationen einschließen und/oder in dieselben aufgebrochen werden.

Wenn einige Aspekte in den vorhergehenden Abschnitten im Zusammenhang mit einer Vorrichtung oder einem System beschrieben wurden, sind diese Aspekte auch als eine Beschreibung des entsprechenden Verfahrens zu verstehen. Dabei kann beispielsweise ein Block, eine Vorrichtung oder ein funktionaler Aspekt der Vorrichtung oder des Systems einem Merkmal, etwa einem Verfahrensschritt, des entsprechenden Verfahrens entsprechen. Entsprechend dazu sind Aspekte, die im Zusammenhang mit einem Verfahren beschrieben werden, auch als eine Beschreibung eines entsprechenden Blocks, eines entsprechenden Elements, einer Eigenschaft oder eines funktionalen Merkmals einer entsprechenden Vorrichtung oder eines entsprechenden Systems zu verstehen.

Die Erfindung wird durch die beigefügten Ansprüche definiert.

## Patentansprüche

1. Ultraschallsystem (600, 700, 800), umfassend:
eine Mehrzahl an modularen Ultraschallgeräten (610-1, ... 610-5, 710-1, 710-2, 810-1, ... 810-5); und
eine Vorrichtung (620, 720, 730, 740) zur Auswertung von Messdaten,
wobei die Mehrzahl an modularen Ultraschallgeräten (610-1, ... 610-5, 710-1, 710-2, 810-1, ... 810-5) ausgebildet sind, jeweilige Messdaten direkt oder durch Weiterleitung über zumindest ein weiteres der Mehrzahl an modularen Ultraschallgeräten (610-1, ... 610-5, 710-1, 710-2, 810-1, ... 810-5) zu einem oder mehreren vorbestimmten modularen Ultraschallgeräten der Mehrzahl an modularen Ultraschallgeräten (610-1, ... 610-5, 710-1, 710-2, 810-1, ... 810-5) zu senden, und
wobei das eine oder die mehreren vorbestimmten modularen Ultraschallgeräten (610-2, 610-4, 710-1) ausgebildet ist, die gesammelten Messdaten der Mehrzahl an modularen Ultraschallgeräten (610-1, ... 610-5, 710-1, 710-2, 810-1, ... 810-5) an die Vorrichtung (620, 720, 730, 740) zur Auswertung von Messdaten zu senden,
wobei die Vorrichtung zur Auswertung von Messdaten ausgebildet ist, eine oder mehrere vorbestimmte Kenngrößen basierend auf den Messdaten zu bestimmen, und
wobei die Mehrzahl an modularen Ultraschallgeräten (610-1, ... 610-5, 710-1, 710-2, 810-1, ... 810-5) jeweils folgendes umfassen:
ein Gehäuse (110);
zumindest einen Ultraschallwandler (140), der ausgebildet ist:
basierend auf einem jeweiligen Steuerungssignal Ultraschallwellen zu erzeugen und auszusenden; und
abhängig von empfangenen Ultraschallwellen ein jeweiliges Messsignal zu erzeugen; und
eine Mehrzahl an in dem Gehäuse (110) angeordneten Platinen (120), die über jeweilige Steckverbinder (131, 132) trennbar miteinander verbunden sind, wobei die Mehrzahl an Platinen (120) zumindest folgendes umfassen:
eine erste Platine (122) mit einer Stromversorgungsschaltung (170), die ausgebildet ist, ein jeweiliges Stromversorgungssignal für den zumindest einen Ultraschallwandler und die weiteren Platinen der Mehrzahl an Platinen (120) zu erzeugen;
eine Sendeschaltung (150), die ausgebildet ist, das jeweilige Steuerungssignale für den zumindest einen Ultraschallwandler (140) zu erzeugen; und
eine Empfangsschaltung (160), die ausgebildet ist, das jeweilige Messsignale des zumindest einen Ultraschallwandlers (140) zu verarbeiten,
wobei die Sendeschaltung (150) und die Empfangsschaltung (160) entweder beide auf einer zweiten Platine (123) der Mehrzahl an Platinen (120) ausgebildet sind oder die Sendeschaltung (150) auf der zweiten Platine (123) und die Empfangsschaltung (160) auf einer dritten Platine (124) der Mehrzahl an Platinen (120) ausgebildet ist,
wobei der zumindest eine Ultraschallwandler (140) auf einer vierten Platine (121) der Mehrzahl an Platinen (120) ausgebildet ist oder trennbar mit einer der Mehrzahl an Platinen (120) verbunden ist, und
wobei die Mehrzahl an Platinen (120) ferner eine fünfte Platine umfasst mit einer Sendeempfängerschaltung, die eingerichtet ist:
basierend auf zu übertragenden Daten des mobilen Ultraschallgeräts (100, 300, 400, 500) ein Sendesignal zu erzeugen;
basierend auf einem Empfangssignal Empfangsdaten für das mobile Ultraschallgerät (100, 300, 400, 500) zu bestimmen;
das Sendesignal an eine Antenne des modularen Ultraschallgeräts (100, 300, 400, 500) zur Abstrahlung in eine Umgebung des modularen Ultraschallgeräts (100, 300, 400, 500) anzulegen und/oder das Sendesignal an eine Schnittstelle des mobilen Ultraschallgeräts (100, 300, 400, 500) zur drahtgebundenen Kommunikation mit einem externen Gerät anzulegen und/oder das Sendesignal an die Sendeschaltung (150) weiterzuleiten, um das jeweilige Steuerungssignal für den zumindest einen Ultraschallwandler (140) basierend auf dem Sendesignal zu erzeugen und so die zu übertragenden Daten des mobilen Ultraschallgeräts (100, 300, 400, 500) in die von dem zumindest einen Ultraschallwandler (140) ausgesendeten Ultraschallwellen zu kodieren; und
das Empfangssignal von der Antenne zu empfangen und/oder das Empfangssignal von der Schnittstelle zur drahtgebundenen Kommunikation mit dem externen Gerät zu empfangen und/oder das Empfangssignal von der Empfangsschaltung (160) zu empfangen, wobei die Empfangsschaltung (160) eingerichtet ist, das Empfangssignal aus dem jeweiligen Messsignal des zumindest einen Ultraschallwandlers (140) abzuleiten.

2. Ultraschallsystem (600, 700, 800) nach Anspruch 1, wobei:
die Mehrzahl an Platinen (120) übereinander angeordnet und aufeinanderfolgende Platinen der Mehrzahl an Platinen (120) mittels der Steckverbinder (131, 132) jeweils zusammengesteckt sind; oder
die Mehrzahl an Platinen (120) nebeneinander angeordnet sind.

3. Ultraschallsystem (600, 700, 800) nach Anspruch 1 oder Anspruch 2, wobei das Gehäuse (110) ein Fenster (115) mit gegenüber dem sonstigen Gehäuse (110) erhöhter akustischer Transparenz umfasst, und wobei das Fenster (115) vor dem zumindest einen Ultraschallwandler (140) angeordnet ist.

4. Ultraschallsystem (600, 700, 800) nach einem der Ansprüche 1 bis 3, wobei die Mehrzahl an Platinen (120) ferner folgendes umfasst:
eine sechste Platine (125) mit einer Prozessierschaltung (180), die ausgebildet ist, eine jeweilige Funktionalität der Mehrzahl an Platinen (120) zu erkennen und basierend darauf einen Datenaustausch zwischen zumindest einem Teil der Mehrzahl an Platinen (120) zu steuern.

5. Ultraschallsystem (600, 700, 800) nach Anspruch 4, wobei die Prozessierschaltung (180) ferner ausgebildet ist, eine oder mehrere vorbestimmte Kenngrößen basierend auf dem durch die Empfangsschaltung (160) verarbeiteten jeweiligen Messsignal des zumindest einen Ultraschallwandlers (140) zu bestimmen, insbesondere unter Verwendung eines mittels maschinellen Lernens trainierten Modells.

6. Ultraschallsystem (600, 700, 800) nach einem der Ansprüche 1 bis 5, wobei die Mehrzahl an Platinen (120) ferner folgendes umfasst:
eine siebte Platine (126) mit einem Datenspeicher (185), der ausgebildet ist, das jeweilige Messsignal des zumindest einen Ultraschallwandlers (140) nach der Verarbeitung durch die Empfangsschaltung (160) zu speichern.

7. Ultraschallsystem (600, 700, 800) nach einem der Ansprüche 1 bis 6, wobei die Mehrzahl an Platinen (120) ferner folgendes umfasst:
eine achte Platine mit einer Positionserfassungsschaltung, die ausgebildet ist, eine absolute Position des modularen Ultraschallgeräts (100, 300, 400, 500) und/oder eine relative Position des modularen Ultraschallgeräts (100, 300, 400, 500) in Bezug auf ein vorbestimmtes Objekt zu bestimmen.

8. Ultraschallsystem (600, 700, 800) nach einem der Ansprüche 1 bis 7, wobei die Empfangsschaltung (160) ausgebildet ist, das jeweilige Messsignal des zumindest einen Ultraschallwandlers (140) bei der Verarbeitung zumindest zu verstärken und zu digitalisieren.

9. Ultraschallsystem (600, 700, 800) nach einem der Ansprüche 1 bis 8, wobei:
die zweite Platine (122) ferner einen mit der Stromversorgungsschaltung (170) gekoppelten Akkumulator (175) umfasst, und wobei die Stromversorgungsschaltung (170) ausgebildet ist, das jeweilige Stromversorgungssignal basierend auf in dem Akkumulator (175) gespeicherter Energie zu erzeugen; oder
in das Gehäuse (110) eine Buchse für die Verbindung mit einem Ladekabel integriert ist, und wobei die Stromversorgungsschaltung (170) ausgebildet ist, das jeweilige Stromversorgungssignal basierend auf an der Buchse empfangener elektrischer Energie zu erzeugen.

10. Ultraschallsystem (600, 700, 800) nach einem der Ansprüche 1 bis 9, wobei in das Gehäuse (110) ein Befestigungselement zur Befestigung des modularen Ultraschallgeräts (100, 300, 400, 500) an einem Prüfobjekt integriert ist.

11. Ultraschallsystem (600, 700, 800) nach einem der Ansprüche 1 bis 10, wobei die Mehrzahl an modularen Ultraschallgeräten (610-1, ... 610-5, 710-1, 710-2, 810-1, ... 810-5) jeweils ferner einen Energiewandler umfassen, der eingerichtet ist, Umgebungsenergie aus der Umgebung des modularen Ultraschallgeräts (100, 300, 400, 500) in elektrische Energie zu wandeln, und wobei die Stromversorgungsschaltung (170) ausgebildet ist, das jeweilige Stromversorgungssignal basierend auf der durch den Energiewandler bereitgestellten elektrischen Energie zu erzeugen.

12. Ultraschallsystem (600, 700, 800) nach einem der Ansprüche 1 bis 11, wobei die Mehrzahl an modularen Ultraschallgeräten (610-1, ... 610-5, 710-1, 710-2, 810-1, ... 810-5) jeweils ferner umfassend eine in das Gehäuse (110) integrierte Benutzerschnittstelle umfassen, die ausgebildet ist:
optisch und/oder akustisch das modulare Ultraschallgerät (100, 300, 400, 500) betreffende Informationen an einen Benutzer auszugeben; und/oder
eine Benutzereingabe des Benutzers zu empfangen.

13. Ultraschallsystem (600, 700, 800) nach einem der Ansprüche 1 bis 12, wobei die Vorrichtung (620, 720, 730, 740) zur Auswertung von Messdaten ausgebildet ist, die eine oder die mehreren vorbestimmten Kenngrößen unter Verwendung eines mittels maschinellen Lernens trainierten Modells aus den Messdaten zu bestimmen.

14. Verfahren (1000) zur Modifikation eines der Mehrzahl an modularen Ultraschallgeräten in dem Ultraschallsystem gemäß einem der Ansprüche 1 bis 13, das Verfahren umfassend:
zerstörungsfreies Trennen (1002) einer der Mehrzahl an Platinen von den übrigen Platinen der Mehrzahl an Platinen durch Lösen zumindest eines Teils der Steckverbinder; und
trennbares Verbinden (1004) einer neuen Platine mit den übrigen Platinen der Mehrzahl an Platinen mittels eines oder mehrerer Steckverbinder.

15. Verfahren (1000) nach Anspruch 14, wobei die neue Platine die gleiche Funktionalität wie die eine der Mehrzahl an Platinen, die von den übrigen Platinen der Mehrzahl an Platinen durch Lösen zumindest eines Teils der Steckverbinder getrennt wird, aufweist.

## Claims

1. An ultrasound system (600, 700, 800), comprising:
a plurality of modular ultrasound devices (610-1, ... 610-5, 710-1, 710-2, 810-1, ... 810-5); and
a device (620, 720, 730, 740) for evaluating measurement data,
wherein the plurality of modular ultrasound devices (610-1, ... 610-5, 710-1, 710-2, 810-1, ... 810-5) are configured to transmit respective measurement data directly or by forwarding via at least one further of the plurality of modular ultrasound devices (610-1, ... 610-5, 710-1, 710-2, 810-1, ... 810-5) to one or more predetermined modular ultrasound devices of the plurality of modular ultrasound devices (610-1, ... 610-5, 710-1, 710-2, 810-1, ... 810-5), and
wherein the one or more predetermined modular ultrasound devices (610-2, 610-4, 710-1) are configured to transmit the collected measurement data of the plurality of modular ultrasound devices (610-1, ... 610-5, 710-1, 710-2, 810-1, ... 810-5) to the device (620, 720, 730, 740) for evaluating measurement data,
wherein the device for evaluating measurement data is configured to determine one or more predetermined characteristic variables based on the measurement data, and
wherein the plurality of modular ultrasound devices (610-1, ... 610-5, 710-1, 710-2, 810-1, ... 810-5) each comprise the following:
a housing (110);
at least one ultrasound transducer (140) configured to:
generate and transmit ultrasound waves based on a respective control signal; and
generate a respective measurement signal depending on received ultrasound waves; and
a plurality of circuit boards (120) arranged in the housing (110), which are separably connected to one another via respective plug connectors (131, 132), wherein the plurality of circuit boards (120) comprise at least the following:
a first circuit board (122) with a power supply circuit (170) configured to generate a respective power supply signal for the at least one ultrasound transducer and the further circuit boards of the plurality of circuit boards (120);
a transmission circuit (150) configured to generate the respective control signals for the at least one ultrasound transducer (140); and
a reception circuit (160) configured to process the respective measurement signals of the at least one ultrasound transducer (140),
wherein the transmission circuit (150) and the reception circuit (160) are either both formed on a second circuit board (123) of the plurality of circuit boards (120) or the transmission circuit (150) is formed on the second circuit board (123) and the reception circuit (160) is formed on a third circuit board (124) of the plurality of circuit boards (120),
wherein the at least one ultrasound transducer (140) is formed on a fourth circuit board (121) of the plurality of circuit boards (120) or is separably connected to one of the plurality of circuit boards (120), and
wherein the plurality of circuit boards (120) further comprises a fifth circuit board with a transceiver circuit configured to:
generate a transmission signal based on data of the mobile ultrasound device (100, 300, 400, 500) to be transmitted;
determine reception data for the mobile ultrasound device (100, 300, 400, 500) based on a reception signal;
apply the transmission signal to an antenna of the modular ultrasound device (100, 300, 400, 500) for radiation into an environment of the modular ultrasound device (100, 300, 400, 500) and/or apply the transmission signal to an interface of the mobile ultrasound device (100, 300, 400, 500) for wired communication with an external device and/or forward the transmission signal to the transmission circuit (150) to generate the respective control signal for the at least one ultrasound transducer (140) based on the transmission signal and thus encode the data of the mobile ultrasound device (100, 300, 400, 500) to be transmitted into the ultrasound waves transmitted by the at least one ultrasound transducer (140); and
receive the reception signal from the antenna and/or receive the reception signal from the interface for wired communication with the external device and/or receive the reception signal from the reception circuit (160), wherein the reception circuit (160) is configured to derive the reception signal from the respective measurement signal of the at least one ultrasound transducer (140).

2. The ultrasound system (600, 700, 800) according to claim 1, wherein:
the plurality of circuit boards (120) are arranged one above the other and successive circuit boards of the plurality of circuit boards (120) are each plugged together by means of the plug connectors (131, 132); or
the plurality of circuit boards (120) are arranged next to one another.

3. The ultrasound system (600, 700, 800) according to claim 1 or claim 2, wherein the housing (110) comprises a window (115) with increased acoustic transparency compared to the rest of the housing (110), and wherein the window (115) is arranged in front of the at least one ultrasound transducer (140).

4. The ultrasound system (600, 700, 800) according to any one of claims 1 to 3, wherein the plurality of circuit boards (120) further comprises the following:
a sixth circuit board (125) with a processing circuit (180) configured to recognize a respective functionality of the plurality of circuit boards (120) and based thereon to control a data exchange between at least a part of the plurality of circuit boards (120).

5. The ultrasound system (600, 700, 800) according to claim 4, wherein the processing circuit (180) is further configured to determine one or more predetermined characteristic variables based on the respective measurement signal of the at least one ultrasound transducer (140) processed by the reception circuit (160), in particular using a model trained by machine learning.

6. The ultrasound system (600, 700, 800) according to any one of claims 1 to 5, wherein the plurality of circuit boards (120) further comprises the following:
a seventh circuit board (126) with a data memory (185) configured to store the respective measurement signal of the at least one ultrasound transducer (140) after processing by the reception circuit (160).

7. The ultrasound system (600, 700, 800) according to any one of claims 1 to 6, wherein the plurality of circuit boards (120) further comprises the following:
an eighth circuit board with a position detection circuit configured to determine an absolute position of the modular ultrasound device (100, 300, 400, 500) and/or a relative position of the modular ultrasound device (100, 300, 400, 500) with respect to a predetermined object.

8. The ultrasound system (600, 700, 800) according to any one of claims 1 to 7, wherein the reception circuit (160) is configured to at least amplify and digitize the respective measurement signal of the at least one ultrasound transducer (140) during processing.

9. The ultrasound system (600, 700, 800) according to any one of claims 1 to 8, wherein:
the second circuit board (122) further comprises an accumulator (175) coupled to the power supply circuit (170), and wherein the power supply circuit (170) is configured to generate the respective power supply signal based on energy stored in the accumulator (175); or
a socket for connection to a charging cable is integrated into the housing (110), and wherein the power supply circuit (170) is configured to generate the respective power supply signal based on electrical energy received at the socket.

10. The ultrasound system (600, 700, 800) according to any one of claims 1 to 9, wherein a fastening element for fastening the modular ultrasound device (100, 300, 400, 500) to a test object is integrated into the housing (110).

11. The ultrasound system (600, 700, 800) according to any one of claims 1 to 10, wherein the plurality of modular ultrasound devices (610-1, ... 610-5, 710-1, 710-2, 810-1, ... 810-5) each further comprise an energy converter configured to convert ambient energy from the surroundings of the modular ultrasound device (100, 300, 400, 500) into electrical energy, and wherein the power supply circuit (170) is configured to generate the respective power supply signal based on the electrical energy provided by the energy converter.

12. The ultrasound system (600, 700, 800) according to any one of claims 1 to 11, wherein the plurality of modular ultrasound devices (610-1, ... 610-5, 710-1, 710-2, 810-1, ... 810-5) each further comprise a user interface integrated into the housing (110), which is configured to:
optically and/or acoustically output information relating to the modular ultrasound device (100, 300, 400, 500) to a user; and/or
receive a user input of the user.

13. The ultrasound system (600, 700, 800) according to any one of claims 1 to 12, wherein the device (620, 720, 730, 740) for evaluating measurement data is configured to determine the one or more predetermined characteristic variables from the measurement data using a model trained by machine learning.

14. A method (1000) for modifying one of the plurality of modular ultrasound devices in the ultrasound system according to any one of claims 1 to 13, the method comprising:
non-destructively separating (1002) one of the plurality of circuit boards from the remaining circuit boards of the plurality of circuit boards by detaching at least a part of the plug connectors; and
separably connecting (1004) a new circuit board to the remaining circuit boards of the plurality of circuit boards by means of one or more plug connectors.

15. The method (1000) according to claim 14, wherein the new circuit board has the same functionality as the one of the plurality of circuit boards which is separated from the remaining circuit boards of the plurality of circuit boards by detaching at least a part of the plug connectors.

## Revendications

1. Système à ultrasons (600, 700, 800), comprenant :
une pluralité d'appareils à ultrasons modulaires (610-1, ... 610-5, 710-1, 710-2, 810-1, ... 810-5) ; et
un dispositif (620, 720, 730, 740) pour l'évaluation de données de mesure,
dans lequel la pluralité d'appareils à ultrasons modulaires (610-1, ... 610-5, 710-1, 710-2, 810-1, ... 810-5) sont configurés à envoyer des données de mesure respectives directement ou par l'intermédiaire d'au moins un autre de la pluralité d'appareils à ultrasons modulaires (610-1, ... 610-5, 710-1, 710-2, 810-1, ... 810-5) à un ou plusieurs appareils à ultrasons modulaires prédéterminés de la pluralité d'appareils à ultrasons modulaires (610-1, ... 610-5, 710-1, 710-2, 810-1, ... 810-5), et
dans lequel le ou les appareils à ultrasons modulaires prédéterminés (610-2, 610-4, 710-1) sont configurés à envoyer les données de mesure collectées de la pluralité d'appareils à ultrasons modulaires (610-1, ... 610-5, 710-1, 710-2, 810-1, ... 810-5) au dispositif (620, 720, 730, 740) pour l'évaluation de données de mesure,
dans lequel le dispositif pour l'évaluation de données de mesure est configuré à déterminer une ou plusieurs grandeurs caractéristiques prédéterminées sur la base des données de mesure, et
dans lequel la pluralité d'appareils à ultrasons modulaires (610-1, ... 610-5, 710-1, 710-2, 810-1, ... 810-5) comprennent respectivement ce qui suit :
un boîtier (110) ;
au moins un transducteur à ultrasons (140) qui est configuré à :
générer et émettre des ondes ultrasonores sur la base d'un signal de commande respectif ; et
générer un signal de mesure respectif en fonction d'ondes ultrasonores reçues ; et
une pluralité de cartes de circuit imprimé (120) disposées dans le boîtier (110), lesquelles sont connectées les unes aux autres de manière séparable par le biais de connecteurs enfichables respectifs (131, 132), dans lequel la pluralité de cartes de circuit imprimé (120) comprennent au moins ce qui suit :
une première carte de circuit imprimé (122) avec un circuit d'alimentation électrique (170) qui est configuré à générer un signal d'alimentation électrique respectif pour l'au moins un transducteur à ultrasons et les autres cartes de circuit imprimé de la pluralité de cartes de circuit imprimé (120) ;
un circuit d'émission (150) qui est configuré à générer les signaux de commande respectifs pour l'au moins un transducteur à ultrasons (140) ; et
un circuit de réception (160) qui est configuré à traiter les signaux de mesure respectifs de l'au moins un transducteur à ultrasons (140),
dans lequel le circuit d'émission (150) et le circuit de réception (160) sont soit tous deux configurés sur une deuxième carte de circuit imprimé (123) de la pluralité de cartes de circuit imprimé (120) soit le circuit d'émission (150) est configuré sur la deuxième carte de circuit imprimé (123) et le circuit de réception (160) est configuré sur une troisième carte de circuit imprimé (124) de la pluralité de cartes de circuit imprimé (120),
dans lequel l'au moins un transducteur à ultrasons (140) est configuré sur une quatrième carte de circuit imprimé (121) de la pluralité de cartes de circuit imprimé (120) ou est connecté de manière séparable à l'une de la pluralité de cartes de circuit imprimé (120), et
dans lequel la pluralité de cartes de circuit imprimé (120) comprend en outre une cinquième carte de circuit imprimé avec un circuit émetteur-récepteur qui est configuré à :
générer un signal d'émission sur la base de données à transmettre de l'appareil à ultrasons mobile (100, 300, 400, 500) ;
déterminer des données de réception pour l'appareil à ultrasons mobile (100, 300, 400, 500) sur la base d'un signal de réception ;
appliquer le signal d'émission à une antenne de l'appareil à ultrasons modulaire (100, 300, 400, 500) pour le rayonnement dans un environnement de l'appareil à ultrasons modulaire (100, 300, 400, 500) et/ou appliquer le signal d'émission à une interface de l'appareil à ultrasons mobile (100, 300, 400, 500) pour la communication filaire avec un appareil externe et/ou transmettre le signal d'émission au circuit d'émission (150) afin de générer le signal de commande respectif pour l'au moins un transducteur à ultrasons (140) sur la base du signal d'émission et de coder ainsi les données à transmettre de l'appareil à ultrasons mobile (100, 300, 400, 500) dans les ondes ultrasonores émises par l'au moins un transducteur à ultrasons (140) ; et
recevoir le signal de réception de l'antenne et/ou recevoir le signal de réception de l'interface pour la communication filaire avec l'appareil externe et/ou recevoir le signal de réception du circuit de réception (160), dans lequel le circuit de réception (160) est configuré à dériver le signal de réception du signal de mesure respectif de l'au moins un transducteur à ultrasons (140).

2. Système à ultrasons (600, 700, 800) selon la revendication 1, dans lequel :
la pluralité de cartes de circuit imprimé (120) sont disposées les unes au-dessus des autres et des cartes de circuit imprimé successives de la pluralité de cartes de circuit imprimé (120) sont respectivement enfichées les unes avec les autres au moyen des connecteurs enfichables (131, 132) ; ou
la pluralité de cartes de circuit imprimé (120) sont disposées les unes à côté des autres.

3. Système à ultrasons (600, 700, 800) selon la revendication 1 ou la revendication 2, dans lequel le boîtier (110) comprend une fenêtre (115) avec une transparence acoustique accrue par rapport à l'autre boîtier (110), et dans lequel la fenêtre (115) est disposée devant l'au moins un transducteur à ultrasons (140).

4. Système à ultrasons (600, 700, 800) selon l'une des revendications 1 à 3, dans lequel la pluralité de cartes de circuit imprimé (120) comprend en outre ce qui suit :
une sixième carte de circuit imprimé (125) avec un circuit de traitement (180) qui est configuré à reconnaître une fonctionnalité respective de la pluralité de cartes de circuit imprimé (120) et, sur la base de celle-ci, à commander un échange de données entre au moins une partie de la pluralité de cartes de circuit imprimé (120).

5. Système à ultrasons (600, 700, 800) selon la revendication 4, dans lequel le circuit de traitement (180) est en outre configuré à déterminer une ou plusieurs grandeurs caractéristiques prédéterminées sur la base du signal de mesure respectif de l'au moins un transducteur à ultrasons (140) traité par le circuit de réception (160), en particulier en utilisant un modèle entraîné par apprentissage automatique.

6. Système à ultrasons (600, 700, 800) selon l'une des revendications 1 à 5, dans lequel la pluralité de cartes de circuit imprimé (120) comprend en outre ce qui suit :
une septième carte de circuit imprimé (126) avec une mémoire de données (185) qui est configurée à mémoriser le signal de mesure respectif de l'au moins un transducteur à ultrasons (140) après le traitement par le circuit de réception (160).

7. Système à ultrasons (600, 700, 800) selon l'une des revendications 1 à 6, dans lequel la pluralité de cartes de circuit imprimé (120) comprend en outre ce qui suit :
une huitième carte de circuit imprimé avec un circuit de détection de position qui est configuré à déterminer une position absolue de l'appareil à ultrasons modulaire (100, 300, 400, 500) et/ou une position relative de l'appareil à ultrasons modulaire (100, 300, 400, 500) par rapport à un objet prédéterminé.

8. Système à ultrasons (600, 700, 800) selon l'une des revendications 1 à 7, dans lequel le circuit de réception (160) est configuré à au moins amplifier et numériser le signal de mesure respectif de l'au moins un transducteur à ultrasons (140) lors du traitement.

9. Système à ultrasons (600, 700, 800) selon l'une des revendications 1 à 8, dans lequel :
la deuxième carte de circuit imprimé (122) comprend en outre un accumulateur (175) couplé au circuit d'alimentation électrique (170), et dans lequel le circuit d'alimentation électrique (170) est configuré à générer le signal d'alimentation électrique respectif sur la base d'énergie stockée dans l'accumulateur (175) ; ou
une prise pour la connexion à un câble de charge est intégrée dans le boîtier (110), et dans lequel le circuit d'alimentation électrique (170) est configuré à générer le signal d'alimentation électrique respectif sur la base d'énergie électrique reçue au niveau de la prise.

10. Système à ultrasons (600, 700, 800) selon l'une des revendications 1 à 9, dans lequel un élément de fixation pour la fixation de l'appareil à ultrasons modulaire (100, 300, 400, 500) à un objet d'essai est intégré dans le boîtier (110).

11. Système à ultrasons (600, 700, 800) selon l'une des revendications 1 à 10, dans lequel la pluralité d'appareils à ultrasons modulaires (610-1, ... 610-5, 710-1, 710-2, 810-1, ... 810-5) comprennent respectivement en outre un convertisseur d'énergie qui est configuré à convertir de l'énergie ambiante provenant de l'environnement de l'appareil à ultrasons modulaire (100, 300, 400, 500) en énergie électrique, et dans lequel le circuit d'alimentation électrique (170) est configuré à générer le signal d'alimentation électrique respectif sur la base de l'énergie électrique fournie par le convertisseur d'énergie.

12. Système à ultrasons (600, 700, 800) selon l'une des revendications 1 à 11, dans lequel la pluralité d'appareils à ultrasons modulaires (610-1, ... 610-5, 710-1, 710-2, 810-1, ... 810-5) comprennent respectivement en outre une interface utilisateur intégrée dans le boîtier (110) qui est configurée à :
émettre optiquement et/ou acoustiquement des informations concernant l'appareil à ultrasons modulaire (100, 300, 400, 500) à un utilisateur ; et/ou
recevoir une entrée utilisateur de l'utilisateur.

13. Système à ultrasons (600, 700, 800) selon l'une des revendications 1 à 12, dans lequel le dispositif (620, 720, 730, 740) pour l'évaluation de données de mesure est configuré à déterminer la ou les grandeurs caractéristiques prédéterminées à partir des données de mesure en utilisant un modèle entraîné par apprentissage automatique.

14. Procédé (1000) de modification d'un de la pluralité d'appareils à ultrasons modulaires dans le système à ultrasons selon l'une des revendications 1 à 13, le procédé comprenant le fait de :
séparer de manière non destructive (1002) l'une de la pluralité de cartes de circuit imprimé des autres cartes de circuit imprimé de la pluralité de cartes de circuit imprimé en détachant au moins une partie des connecteurs enfichables ; et
connecter de manière séparable (1004) une nouvelle carte de circuit imprimé aux autres cartes de circuit imprimé de la pluralité de cartes de circuit imprimé au moyen d'un ou de plusieurs connecteurs enfichables.

15. Procédé (1000) selon la revendication 14, dans lequel la nouvelle carte de circuit imprimé présente la même fonctionnalité que l'une de la pluralité de cartes de circuit imprimé qui est séparée des autres cartes de circuit imprimé de la pluralité de cartes de circuit imprimé en détachant au moins une partie des connecteurs enfichables.
